# EUROPEAN PATENT APPLICATION

(11) **EP 4 800 008 A1**
(43) Date of publication of application: **02.09.2026**
(21) Application number: 24882259.5
(22) Date of filing: 16.10.2024
(51) Int. Cl.: C07D 231/14, A01N 43/54, A01P 3/00, C07D 401/12

(54) **PYRAZOLE DERIVATIVE, OR ACCEPTABLE SALT THEREOF OR N-OXIDE THEREOF, AGRICULTURAL AND HORTICULTURAL CHEMICAL, AND INDUSTRIAL MATERIAL PROTECTANT**

(30) Priority: 23.10.2023 JP 2023182023
(71) Applicant: Kureha Corporation, Chuo-ku Tokyo 103-8552 (JP)
(72) Inventor: ANABUKI, Tomoaki, Tokyo 103-8552 (JP); DOHI, Soya, Tokyo 103-8552 (JP); KOSHIYAMA, Tatsuyuki, Tokyo 103-8552 (JP)
(74) Representative: Zimmermann & Partner Patentanwälte mbB
(86) International application number: PCT/JP2024/036764
(87) International publication number: WO 2025/089146

(57) **Abstract**

The present invention provides a plant disease controlling agent that has an excellent control efficacy against a wide range of plant diseases. Specifically, a pyrazole derivative represented by General Formula (I) below, or an agrochemically acceptable salt thereof or an N-oxide thereof is provided.

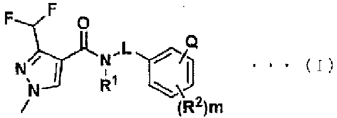

## Description

### TECHNICAL FIELD

The present invention relates to a novel pyrazole derivative, or an acceptable salt thereof or an N-oxide thereof, an agricultural or horticultural chemical, and an industrial material protectant.

### BACKGROUND ART

As agricultural or horticultural chemicals, chemicals containing pyrazole derivatives have been investigated. For example, techniques using a pydiflumetofen compound for agricultural or horticultural use have been known as typical techniques (e.g., Patent Documents 1 and 2).

### CITATION LIST

### Patent Document

Patent Document 1: WO 2010/063700
Patent Document 2: WO 2014/177514

### SUMMARY OF INVENTION

### Technical Problem

There is a need for a plant disease controlling agent that has an excellent control efficacy against a wide range of plant diseases. Under such circumstances, for pyrazole derivatives, there is room for investigating a novel compound having superior effect.

The present invention has been completed in light of the problems described above. An objective of the present invention is to provide a compound that meets the above requirements.

### SOLUTION TO PROBLEM

As a result of diligent research to solve the above problems, the present inventors have found that a pyrazole derivative represented by General Formula (I) below have excellent activity, and thus have completed the present invention.

The pyrazole derivative according to an aspect of the present invention is a pyrazole derivative represented by General Formula (I), or an agrochemically acceptable salt thereof or an N-oxide thereof: 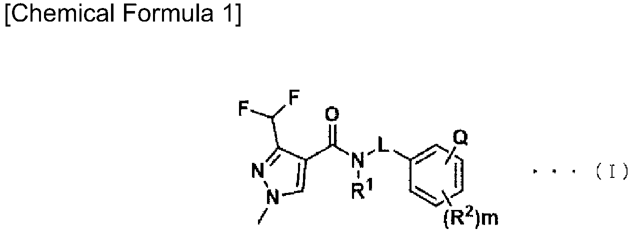 where, in General Formula (I),
Q is X-A-B;
X is a C₂-C₈ alkynylene group, a C₂-C₈ alkenylene group, or a C₁-C₈ alkylene group;
A is (i) -O-, -SO₂-, -COO-, or -NR^{Q}-, or (ii) a single bond or a linear C₁-C₄ alkylene group,
R^{Q} is hydrogen or a C₁-C₄ alkyl group,
B is a phenyl group, a benzyl group, a pyridyl group, a naphthyl group, a quinolinyl group, a pyridylmethyl group, or a tetrahydropyranyl group;
B is unsubstituted or substituted with n R^{B} substituents,
when A is (i), R^{B} is a halogen group, a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group;
when A is (ii), R^{B} is a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group;
n is 1, 2, 3, or 4, and when n is 2 or more, the plurality of R^{B} substituents are each independently selected;
R² is a halogen group, a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group; m is 0, 1, 2, 3, or 4, and when m is 2 or more, the plurality of R² substituents are each independently selected;
R¹ is hydrogen, a C₁-C₄ alkyl group, a C₃-C₆ cycloalkyl group, a C₁-C₄ alkoxy group, a C₁-C₄ haloalkyl group, or a hydroxy group; and
L is a C₁-C₆ alkylene group.

### ADVANTAGEOUS EFFECTS OF INVENTION

The pyrazole derivative according to an embodiment of the present invention exhibits an excellent control efficacy against a wide range of plant diseases. Therefore, the agent containing the pyrazole derivative according to an embodiment of the present invention as an active ingredient exhibits a high control efficacy against a wide variety of plant diseases.

### DESCRIPTION OF EMBODIMENTS

Unless otherwise noted in the present specification, the expression "(from) A to B" representing a numerical range means a range including the A and B themselves, that is, "A or greater and B or less".

### 1. Pyrazole Derivative

The pyrazole derivative according to an embodiment of the present invention is a pyrazole derivative represented by Formula (I) below (hereinafter, also referred to as pyrazole derivative (I)) or an agrochemically acceptable salt thereof or an N-oxide thereof.

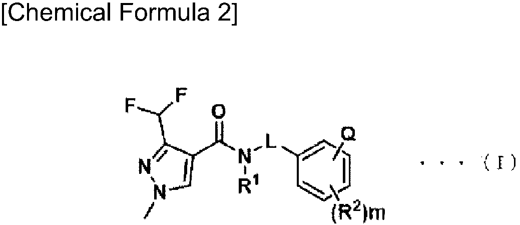

Each substituent in General Formula (I) above are described below in detail.

Q is X-A-B. The bonding position of Q in the phenyl group is not limited; however, Q is preferably bonded at a para-position, that is 4-position, with respect to the bonding position of L. That is, examples of a preferred aspect of the pyrazole derivative (I) include a pyrazole derivative represented by General Formula (II) below or an agrochemically acceptable salt thereof or an N-oxide thereof.

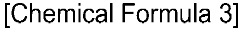

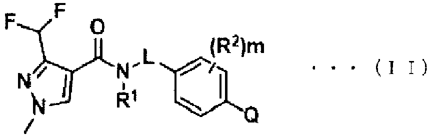

Each substituent of the pyrazole derivative (II) has the same configuration described as configuration of each substituent of the pyrazole derivative (I) described below in detail.

X is a C₂-C₈ alkynylene group, a C₂-C₈ alkenylene group, or a C₁-C₈ alkylene group.

The C₂-C₈ alkynylene group is a linear or branched alkynylene group having from 2 to 8 carbon atoms, and examples thereof include an ethynylene group, a 2-propynylene group, a 1-propynylene group, a 3-butynylene group, a 2-butynylene group, a 1-butynylene group, a 1-methyl-2-propynylene group, a 2-pentynylene group, a 3-pentynylene group, a 4-pentynylene group, a 1,1-dimethyl-2-propynylene group, a 1-hexynylene group, a 5-hexynylene group, a 1-heptynylene group, a 6-heptynylene group, a 1-octynylene group, and a 7-octynylene group.

The C₂-C₈ alkenylene group is a linear or branched alkenylene group having from 2 to 8 carbon atoms, and examples thereof include an ethenylene group, a 2-propenylene group, a 1-propenylene group, a 1-methylethenylene group, a 3-butenylene group, a 2-butenylene group, a 1-butenylene group, a 1-methyl-1-propenylene group, a 2-methyl-1-propenylene group, a 1-methyl-2-propenylene group, a 2-methyl-2-propenylene group, a 1,3-butadienylene group, a 4-pentenylene group, a 3-pentenylene group, a 2-pentenylene group, a 1-pentenylene group, a 1-methyl-1-butenylene group, a 1-methyl-2-butenylene group, a 1-methyl-3-butenylene group, a 3-methyl-1-butenylene group, a 1,1-dimethyl-2-propenylene group, a 1,2-dimethyl-2-propenylene group, a 2-methyl-2-butenylene group, a 3-methyl-2-butenylene group, a 1,2-dimethyl-1-propenylene group, a 2-methyl-3-butenylene group, a 3-methyl-3-butenylene group, a 1,3-pentadienylene group, a 1-ethyl-2-propenylene group, a 1-vinyl-2-propenylene group, a 5-hexenylene group, a 1-hexenylene group, a 6-heptenylene group, a 1-heptenylene group, a 7-octenylene group, and a 1-octenylene group.

The C₁-C₈ alkylene group is a linear or branched alkylene group having from 1 to 8 carbon atoms, and examples thereof include a methylene group, an ethylene group, a propylene group, a 1-methylethylene group, a butylene group, a 2-methylpropylene group, a 1-methylpropylene group, a 1,1-dimethylethylene group, a pentylene group, a 3-methylbutylene group, a 2-methylbutylene group, a 1-methylbutylene group, a 2,2-dimethylpropylene group, a 1,2-dimethylpropylene group, a 1,1-dimethylpropylene group, a 1-ethylpropylene group, a hexylene group, a 4-methylpentylene group, a 3-methylpentylene, a 2-methylpentylene group, a 1-methylpentylene group, a 3,3-dimethylbutylene group, a 2,2-dimethylbutylene group, a 1,1-dimethylbutylene group, a 1,2-dimethylbutylene group, a 1,3-dimethylbutylene group, a 2,3-dimethylbutylene group, a 1-ethylbutylene group, a 2-ethylbutylene group, a 1,1,2-trimethylpropylene group, a 1,2,2-trimethylbutylene group, a 1-ethyl-1-methylpropylene group, a 1-ethyl-2-methylpropylene group, a heptylene group, and an octylene group.

From the viewpoint of the control efficacy against plant diseases, X is preferably a C₂-C₈ alkynylene group. For example, X may be a C₂-C₄ alkynylene group. For example, X may be an ethynylene group, a 1-propynylene group, a 1-butynylene group, a (Z)-1-propenylene group, or a propylene group, and is preferably an ethynylene group, a 1-propynylene group, or a 1-butynylene group.

A is (i) -O-, -SO₂-, -COO-, or -NR^{Q}-, or (ii) a single bond or a linear C₁-C₄ alkylene group. From the viewpoint of the control efficacy against plant diseases, A is preferably (i) -O-, -SO₂-, or -NR^{Q}-, or (ii) a single bond. A is particularly preferably -O-.

The C₁-C₄ alkylene group is a linear or branched alkylene group having from 1 to 4 carbon atoms, and examples thereof include a methylene group, an ethylene group, a propylene group, a 1-methylethylene group, a butylene group, a 2-methylpropylene group, a 1-methylpropylene group, and a 1,1-dimethylethylene group.

R^{Q} is hydrogen or a C₁-C₄ alkyl group.

The C₁-C₄ alkyl group is a linear or branched alkyl group having from 1 to 4 carbon atoms, and examples thereof include a methyl group, an ethyl group, a propyl group, a 1-methylethyl group, a butyl group, a 2-methylpropyl group, a 1-methylpropyl group, and a 1,1-dimethylethyl group.

R^{Q} is preferably a C₁-C₄ alkyl group. For example, R^{Q} may be a methyl group.

B is a phenyl group, a benzyl group, a pyridyl group, a naphthyl group, a quinolinyl group, a pyridylmethyl group, or a tetrahydropyranyl group. The group represented by B is unsubstituted or substituted with n R^{B} substituents. From the viewpoint of the control efficacy against plant diseases, B is preferably a phenyl group, a benzyl group, or a pyridyl group.

When A is (i), R^{B} is a halogen group, a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group.

The halogen group is a chloro group, a bromo group, an iodo group, or a fluoro group.

The C₁-C₄ alkoxy group is a linear or branched alkoxy group having from 1 to 4 carbon atoms, and examples thereof include a methoxy group, an ethoxy group, a propoxy group, a 1-methylethoxy group, a butoxy group, 2-methylpropoxy group, 1-methylpropoxy group, and 1,1-dimethylethoxy group.

The C₁-C₄ haloalkyl group represents a group, in which at least one hydrogen atom of a linear or branched alkyl group having from 1 to 4 carbon atoms is substituted with a halogen atom, and examples thereof include a fluoromethyl group, a chloromethyl group, a bromomethyl group, a difluoromethyl group, a dichloromethyl group, a chlorofluoromethyl group, a trifluoromethyl group, a trichloromethyl group, a tribromomethyl group, a chlorodifluoromethyl group, a dichlorofluoromethyl group, a 1-fluoroethyl group, a 1-chloroethyl group, a 1-bromoethyl group, a 2-fluoroethyl group, a 2,2-difluoroethyl group, a 2-chloro-2-fluoroethyl group, a 2,2,2-trifluoroethyl group, a 2,2,2-trichloroethyl group, a 2-chloro-2,2-difluoroethyl group, a 2,2-dichloro-2-fluoroethyl group, a pentafluoroethyl group, a 3-fluoropropyl group, a 2-fluoropropyl group, a 2,2-difluoropropyl group, a 3,3,3-trifluoropropyl group, and a 4-fluorobutyl group.

The C₁-C₄ haloalkoxy group represents a group, in which at least one hydrogen atom of a linear or branched alkoxy group having from 1 to 4 carbon atoms is substituted with a halogen atom, and examples thereof include a fluoromethoxy group, a difluoromethoxy group, a trifluoromethoxy group, a chloromethoxy group, a dichloromethoxy group, a trichloromethoxy group, a chlorofluoromethoxy group, a chlorodifluoromethoxy group, a dichlorofluoromethoxy group, a 2-fluoroethoxy group, a 2-chloroethoxy group, a 2-bromoethoxy group, a 2-iodoethoxy group, a 2,2-difluoroethoxy group, a 2-chloro-2-fluoroethoxy group, a 2,2,2-trifluoroethoxy group, a 2-chloro-2,2-difluoroethoxy group, a 2,2-dichloro-2-fluoroethoxy group, a 2,2,2-trichloroethoxy group, a 2,2,2-tribromoethoxy group, a 2,2,2-triiodoethoxy group, a pentafluoroethoxy group, a pentachloroethoxy group, a 2-fluoropropoxy group, a 3-fluoropropoxy group, a 2,2-difluoropropoxy group, a 2,3-difluoropropoxy group, a 2-chloropropoxy group, a 3-chloropropoxy group, a 2,2-dichloropropoxy group, a 2,3-dichloropropoxy group, a 2-bromopropoxy group, a 3-bromopropoxy group, a 3,3,3-trifluoropropoxy group, a 3,3,3-trichloropropoxy group, a 3,3,3-tribromopropoxy group, a 3,3,3-triiodopropoxy group, a 2,2,3,3,3-pentafluoropropoxy group, a heptafluoropropoxy group, a heptachloropropoxy group, a heptabromopropoxy group, a heptaiodopropoxy group, a 1-fluoromethyl-2-fluoroethoxy group, a 1-chloromethyl-2-chloroethoxy group, a 1-bromomethyl-2-bromoethoxy group, a 4-fluorobutoxy group, a 4-chlorobutoxy group, a 4-bromobutoxy group, a nonafluorobutoxy group, a nonachlorobutoxy group, a nonabromobutoxy group, and a nonaiodobutoxy group.

The C₁-C₄ alkoxy-C₁-C₄ alkyl group represents a group, in which one or two or more hydrogen atoms of a linear or branched alkyl group having from 1 to 4 carbon atoms are substituted with C₁-C₄ alkoxy group(s), and preferably represents a group, in which only one hydrogen atom of the alkyl group is substituted with a C₁-C₄ alkoxy group. Examples thereof include a methoxymethyl group, a methoxyethyl group, a methoxypropyl group, a methoxybutyl group, an ethoxymethyl group, an ethoxyethyl group, an ethoxypropyl group, and an ethoxybutyl group.

From the viewpoint of the control efficacy against plant diseases, when A is (i), R^{B} is preferably a halogen group, a C₁-C₄ alkyl group, a C₁-C₄ alkoxy group, a C₁-C₄ haloalkyl group, a C₁-C₄ haloalkoxy group, or an amino group. Furthermore, R^{B} is particularly preferably a halogen group or a C₁-C₄ alkoxy group. For example, R^{B} may be a methoxy group, a methoxymethyl group, a trifluoromethoxy group, a chloro group, a methyl group, or an amino group. The bonding position of R^{B} is not particularly limited and can be appropriately changed based on the type of B.

When A is (ii), R^{B} is a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group. From the viewpoint of the control efficacy against plant diseases, when A is (ii), R^{B} is preferably a C₁-C₄ alkyl group, a C₁-C₄ alkoxy group, a C₁-C₄ haloalkyl group, a C₁-C₄ haloalkoxy group, or an amino group. For example, R^{B} may be a methoxy group, a methoxymethyl group, or a trifluoromethoxy group. Note that the C₁-C₄ alkyl group, the C₁-C₄ alkoxy group, the C₁-C₄ haloalkyl group, the C₁-C₄ haloalkoxy group, and the C₁-C₄ alkoxy-C₁-C₄ alkyl group are as described above.

n is an integer representing the number of R^{B} substituents and is 1, 2, 3, or 4. When n is 2 or more, the plurality of R^{B} substituents are each independently selected. When A is (i), n is preferably 1 or 2. When A is (ii), n is preferably 1.

R² is a halogen group, a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group. The halogen group, the C₁-C₄ alkyl group, the C₁-C₄ alkoxy group, the C₁-C₄ haloalkyl group, the C₁-C₄ haloalkoxy group, and the C₁-C₄ alkoxy-C₁-C₄ alkyl group are as described above.

R² is preferably a halogen group. For example, R² may be a fluoro group or a chloro group. The bonding position of R² is not particularly limited. For example, the bonding position is preferably 2-position, 3-position, 5-position, or 6-position, and particularly preferably 2-position or 6-position, with respect to the bonding of L in General Formula (I); however, the bonding position is not limited to these.

m is an integer representing the number of R² substituents and is 0, 1, 2, 3, or 4. When m is 2 or more, the plurality of R² substituents are each independently selected. m is preferably 1 or 2.

R¹ is hydrogen, a C₁-C₄ alkyl group, a C₃-C₆ cycloalkyl group, a C₁-C₄ alkoxy group, a C₁-C₄ haloalkyl group, or a hydroxy group. The C₁-C₄ alkyl group, the C₁-C₄ alkoxy group, and the C₁-C₄ haloalkyl group are as described above.

The C₃-C₆ cycloalkyl group is a cyclic alkyl group having from 3 to 6 carbon atoms, and examples thereof include a cyclopropyl group, a cyclobutyl group, a cyclopentyl group, and a cyclohexyl group.

From the viewpoint of the control efficacy against plant diseases, R¹ is preferably a C₁-C₄ alkoxy group. For example, R¹ may be a methyl group, an ethyl group, a hydroxy group, a methoxy group, an ethoxy group, a cyclopropyl group, or a difluoroethyl group.

L is a C₁-C₆ alkylene group. The C₁-C₆ alkylene group is a linear or branched alkylene group having from 1 to 6 carbon atoms, and examples thereof include a methylene group, an ethylene group, a methylmethylene group, a propylene group, a 1-methylethylene group, a butylene group, a 2-methylpropylene group, a 1-methylpropylene group, a 1,1-dimethylethylene group, a pentylene group, a 3-methylbutylene group, a 2-methylbutylene group, a 1-methylbutylene group, a 2,2-dimethylpropylene group, a 1,2-dimethylpropylene group, a 1,1-dimethylpropylene group, a 1-ethylpropylene group, a hexylene group, a 4-methylpentylene group, a 3-methylpentylene group, a 2-methylpentylene group, a 1-methylpentylene group, a 3,3-dimethylbutylene group, a 2,2-dimethylbutylene group, a 1,1-dimethylbutylene group, a 1,2-dimethylbutylene group, a 1,3-dimethylbutylene group, a 2,3-dimethylbutylene group, a 1-ethylbutylene group, a 2-ethylbutylene group, a 1,1,2-trimethylpropylene group, a 1,2,2-trimethylbutylene group, a 1-ethyl-1-methylpropylene group, and a 1-ethyl-2-methylpropylene group.

For example, L may be -CH₂-, -CH₂CH₂-, -CH(CH₃)-, -CH(CH₃)CH₂-, and -CH(CH₃)CH₂CH₂-.

Among these, L is preferably a branched C₁-C₆ alkylene group.

The pyrazole derivative (I) according to the present embodiment exhibits the control efficacy against a wide range of plant diseases. Here, "plant disease" refers to abnormality, such as deterioration of physiological functions of a plant and growth insufficiency, caused by insect pests and pathogenic microorganisms. The agricultural or horticultural chemical in the present embodiment is particularly suitable for controlling plant diseases caused by pathogenic microorganisms because the agricultural or horticultural chemical exhibits the antifungal effect against a wide range of pathogenic fungi.

The pyrazole derivative (I) may be, for example, the following compound.
In General Formula (I),
X may be a C₂-C₄ alkynylene group;
A may be (i) -O-, -SO₂-, -NCH₃-, or (ii) a single bond;
B may be a phenyl group, a benzyl group, or a pyridyl group;
R² may be -F or -Cl;
when A is (i), R^{B} may be a methoxy group, a methoxymethyl group, a trifluoromethoxy group, a chloro group, a methyl group, or an amino group;
when A is (ii), R^{B} may be a methoxy group, a methoxymethyl group, or a trifluoromethoxy group;
R¹ may be a methyl group, an ethyl group, a hydroxy group, a methoxy group, an ethoxy group, a cyclopropyl group, or a difluoroethyl group; and
L may be -CH₂-, -CH₂CH₂-, -CH(CH₃)-, -CH(CH₃)CH₂-, or -CH(CH₃)CH₂CH₂-.

Furthermore, for example, the pyrazole derivative (I) may be the following compound.

In General Formula (I),
X may be a propynylene group;
A may be -O-;
B may be a phenyl group, a benzyl group, or a pyridyl group;
R² may be -Cl;
m may be 1 or 2;
R¹ may be a methoxy group; and
L may be -CH(CH₃)CH₂-.

Table 1 below lists pyrazole derivatives as examples of particularly preferable pyrazole derivatives (I). X, A, B, R¹, L, R² in Table 1 below respectively correspond to X, A, B, R¹, L, R² of Formula (I) above. For example, "2-Cl" in R² represents that Cl is bonded at 2-position with respect to the bonding of L. "2,6-diCl" represents Cl substituents are each bonded to 2-position and 6-position with respect to the bonding of L. Furthermore, "-" in A represents a single bond.

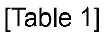

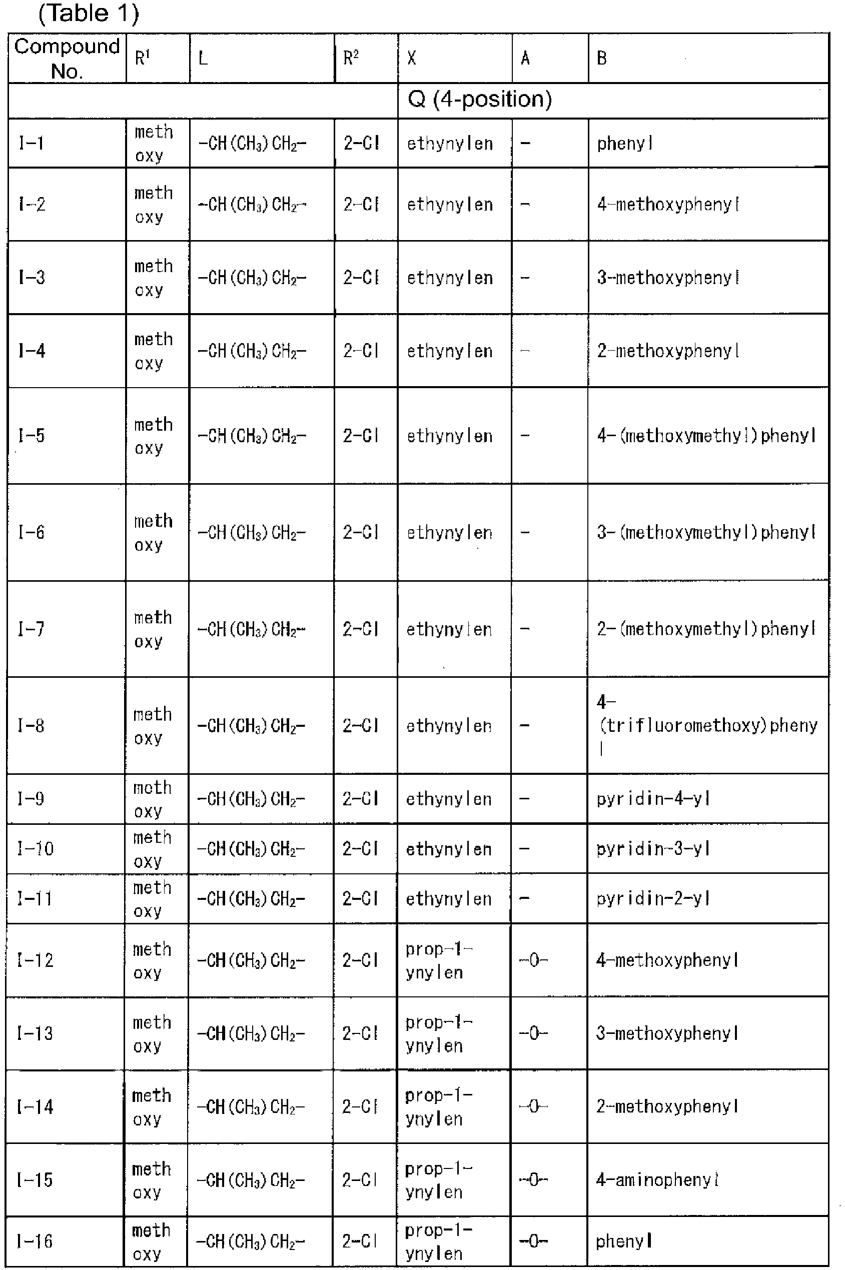

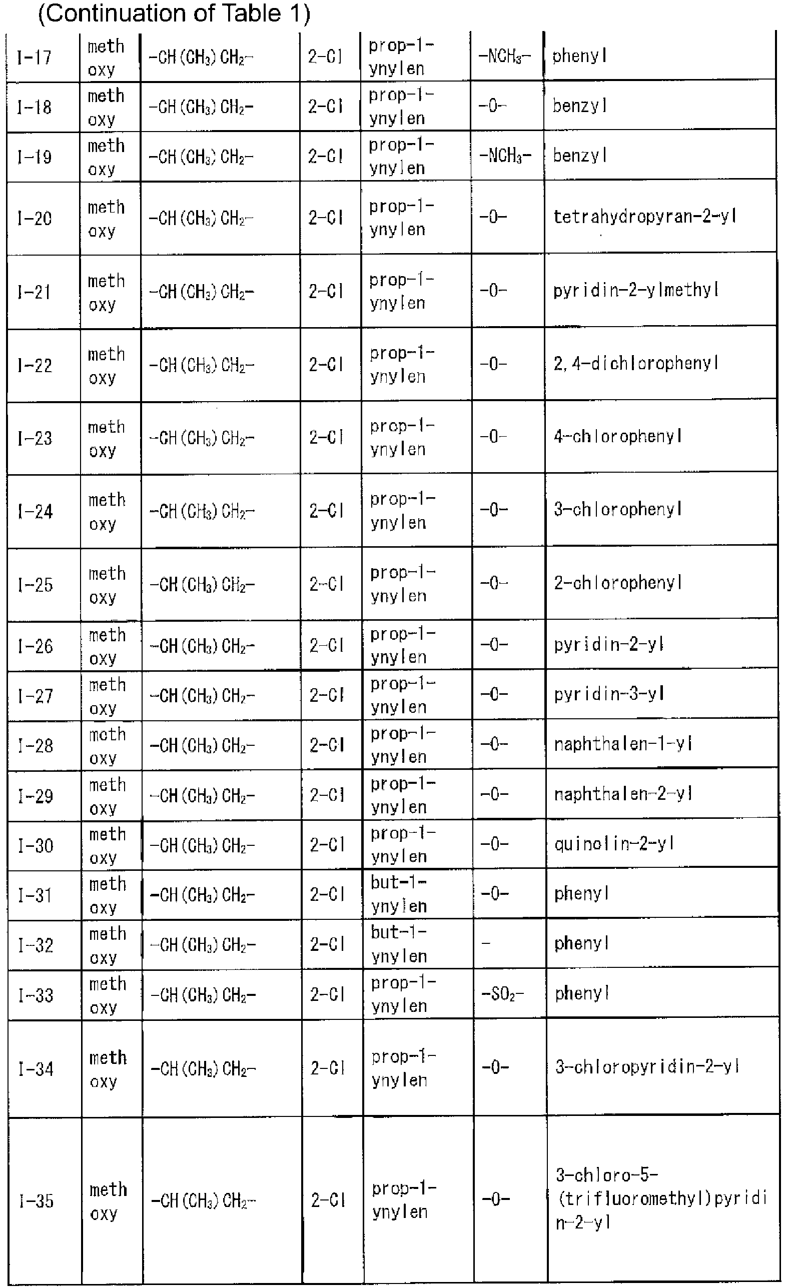

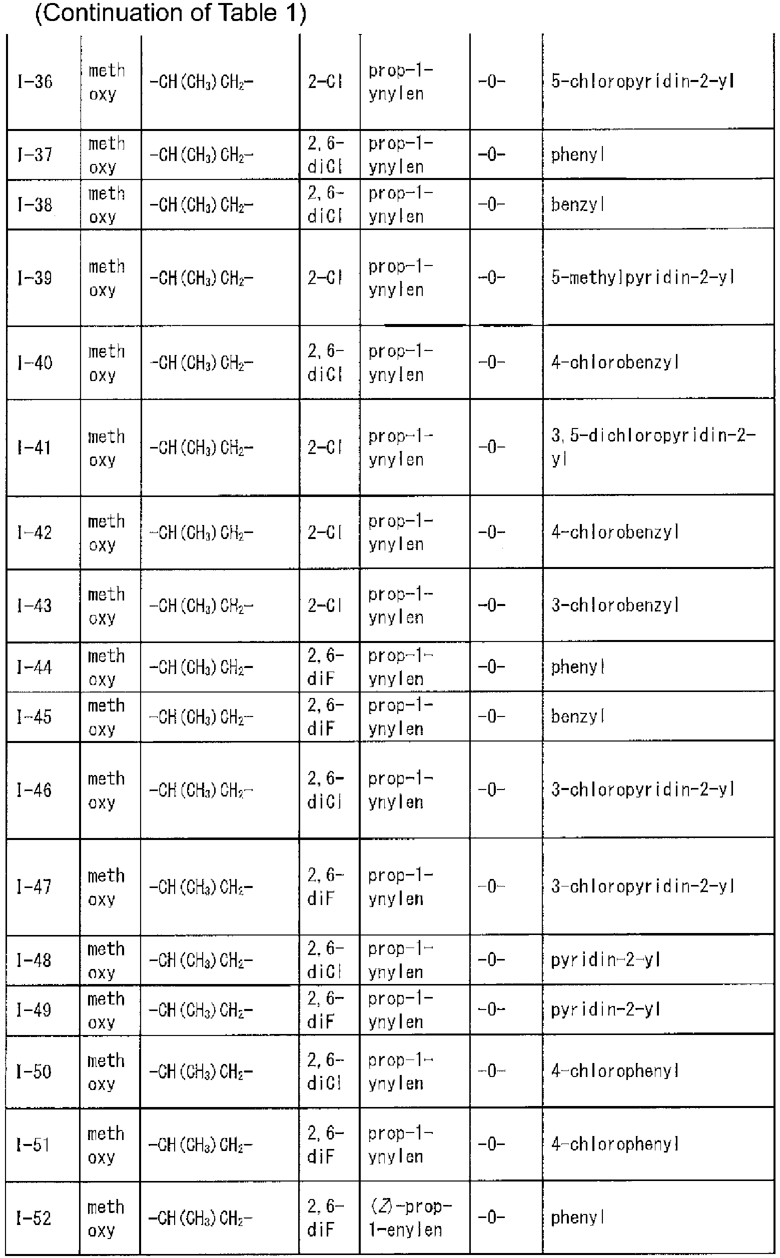

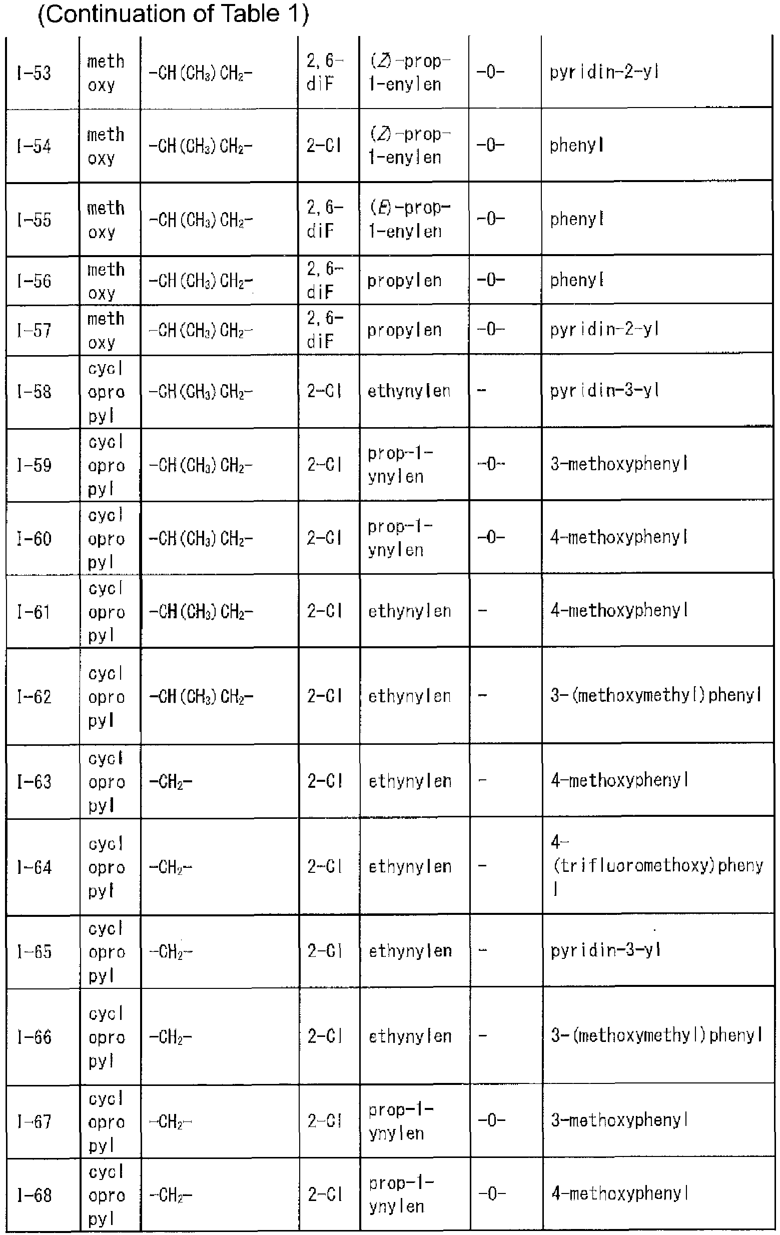

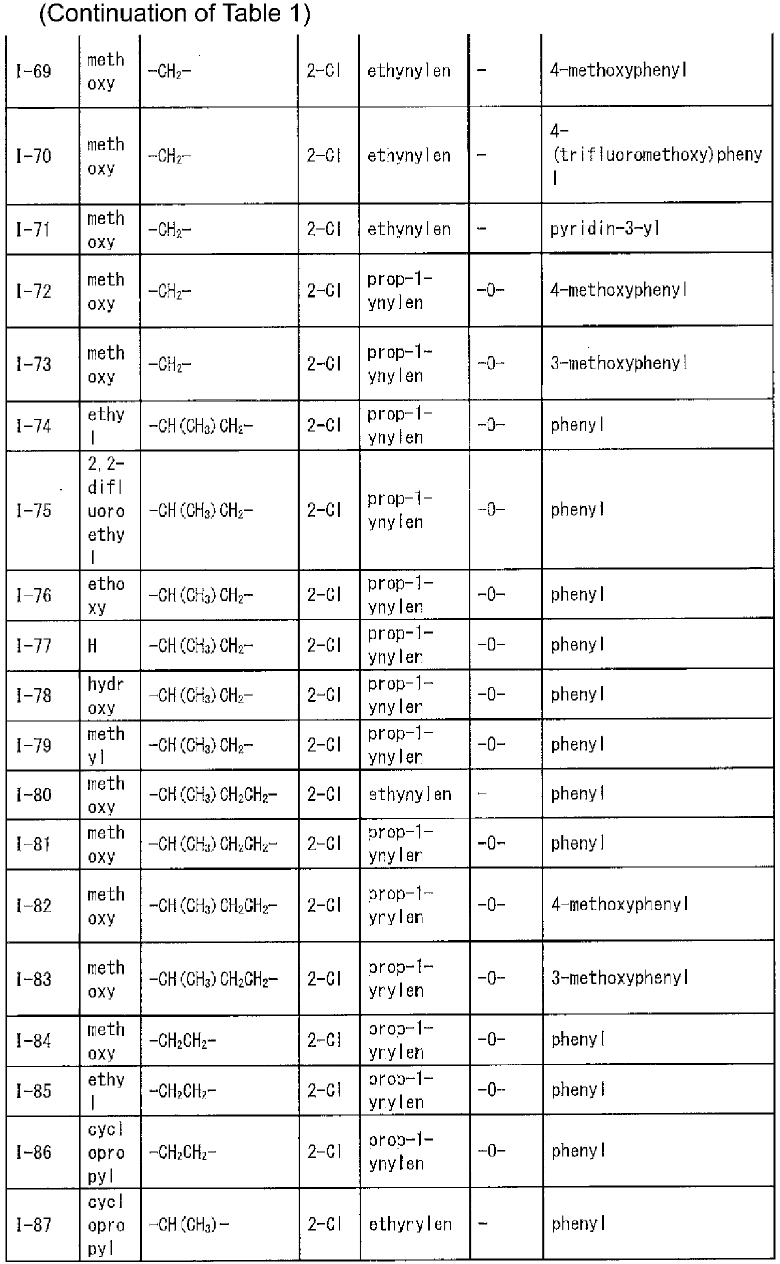

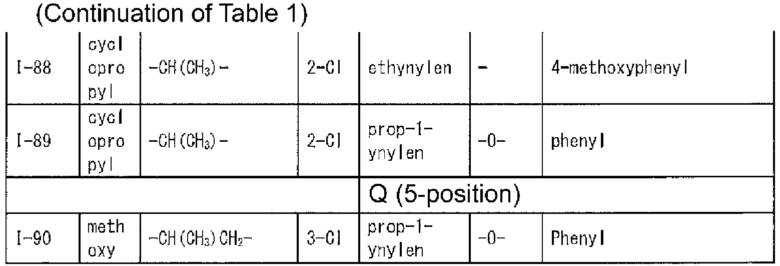

With the compounds represented by the pyrazole derivative (I) of the present embodiment, examples of acid addition salts that can be formed by an ordinary method include a salt of hydrohalic acid, such as hydrofluoric acid, hydrochloric acid, hydrobromic acid, and hydriodic acid; a salt of inorganic acid, such as nitric acid, sulfuric acid, phosphoric acid, chloric acid, and perchloric acid; a salt of sulfonic acid, such as methanesulfonic acid, ethanesulfonic acid, trifluoromethanesulfonic acid, benzenesulfonic acid, and p-toluenesulfonic acid; a salt of carboxylic acid, such as formic acid, acetic acid, propionic acid, trifluoroacetic acid, fumaric acid, tartaric acid, oxalic acid, maleic acid, malic acid, succinic acid, benzoic acid, mandelic acid, ascorbic acid, lactic acid, gluconic acid, and citric acid; and a salt of an amino acid, such as glutamic acid and aspartic acid.

Alternatively, examples of metal salts that can be formed by an ordinary method with the pyrazole derivative (I) of the present embodiment include a salt of an alkali metal, such as lithium, sodium, or potassium; a salt of an alkaline earth metal, such as calcium, barium, or magnesium; or a salt of aluminum.

### 2. Method for Producing Pyrazole Derivative

The pyrazole derivative (I) can be produced by any of the methods described below. Q, X, A, B, R¹, R², m, and L in the following scheme respectively correspond to Q, X, A, B, R¹, R², m, and L in General Formula (I) above. In addition, a compound represented by Formula x (x is a number or an alphabet) is simply referred to as a compound x. For example, a compound represented by Formula a is written as "compound a". Note that in the reaction of the step using a known reaction mechanism, various conditions such as reagents, bases, solvents, and the like that are subjected to the reaction, and temperature are within a range that may be appropriately set by a person skilled in the art based on common general technical knowledge.

### (1) Method 1 for Producing Pyrazole Derivative

A pyrazole derivative can be produced from a compound a obtained by a known technique according to the following synthesis scheme 1. In the synthesis scheme 1, the compound I·A, the compound I·B, and the compound I·C correspond to the pyrazole derivative (I). That is, step 2 and step 3 are optional steps that are performed depending on a target end product. 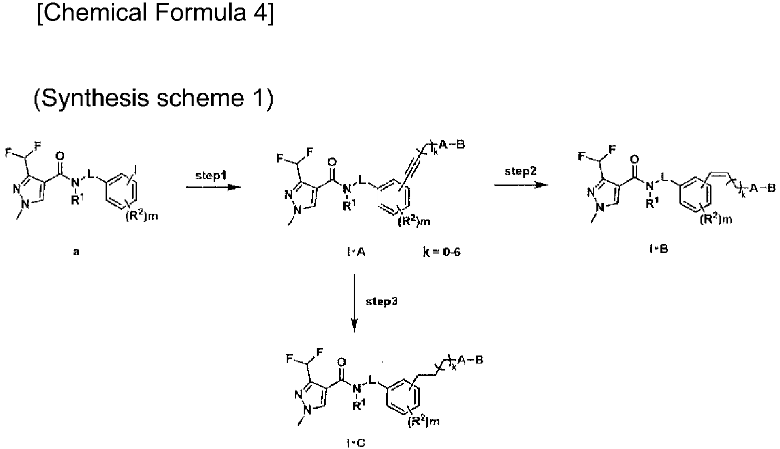 (step 1) A compound I·A is obtained by reacting a compound a with a terminal alkyne in the presence of a palladium catalyst. The terminal alkyne used in step 1 is represented by Formula (III) below.

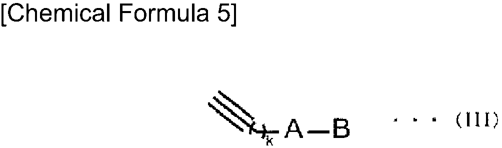

In the compound I·A and Formula (III) above, k is an integer representing the number of carbon atoms and is 1, 2, 3, 4, 5, or 6.
(step 2) The step 2 is a step to obtain a pyrazole derivative (I) in which X is an alkenylene group. Poisoning by a freely chosen catalyst poison such as quinoline or lead acetate is performed, and a calcium carbonate-supported palladium catalyst having a reduced catalytic activity and the obtained compound I·A are reacted, and thus a compound represented by General Formula I·B is obtained.
(step 3) The step 3 is a step to obtain a pyrazole derivative (I) in which X is an alkylene group. An activated carbon-supported palladium catalyst and the obtained compound I·B are reacted, and thus a compound I·C is obtained.

### (1) Method 2 for Producing Pyrazole Derivative

A pyrazole derivative can be produced from a compound a obtained by a known technique according to the following synthesis scheme 2. 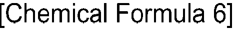 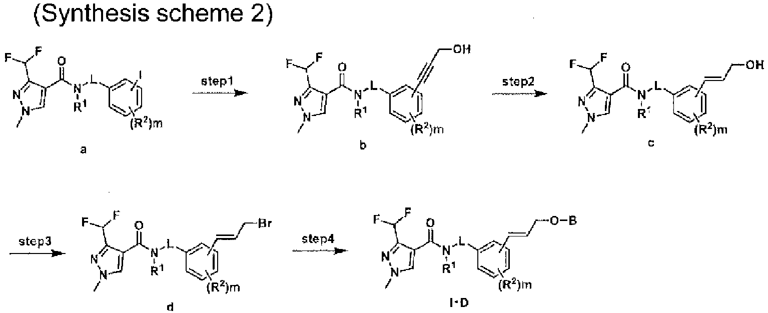
(step 1) A compound b is obtained by reacting a compound a with propargyl alcohol in the presence of a palladium catalyst.
(step 2) A compound c is obtained by reacting a compound b and a freely chosen reducing agent. An example of the freely chosen reducing agent is sodium bis(2-methoxyethoxy)aluminum hydride but the freely chosen reducing agent is not limited to this.
(step 3) A compound d is obtained by converting a hydroxy group of the compound c into a halogen group. Note that, in the scheme, a bromo group is shown as an example of a halogen group of the compound d; however, the halogen group is not limited to this and, for example, may be a chloro group, a bromo group, an iodo group, or a fluoro group. For example, an example of a reaction converting into a bromo group is a reaction using carbon tetrabromide and triphenylphosphine but the reaction is not limited to this.
(step 4) A compound I·D is obtained by substituting the bromo group of the compound d with an alcohol or hydroxy group-containing aromatic compound substituted with B in the presence of a freely chosen base. An example of the freely chosen base is a carbonate such as cesium carbonate but the base is not limited to this.

### 3. Method for Producing N-Oxide of Pyrazole Derivative

The N-oxide can be prepared from the pyrazole derivative (I) by any method.

### 4. Agricultural or Horticultural Chemical

Hereinafter, even when "pyrazole derivative (I)" is simply written, "pyrazole derivative (I)" means all of the pyrazole derivative (I), and the agrochemically acceptable salt of the pyrazole derivative (I) and the N-oxide thereof. Since the pyrazole derivative (I) has a pyrazole group, it forms an acid addition salt of inorganic acids and organic acids, or a metal complex. Therefore, it can be used as an active ingredient of agricultural or horticultural chemical agents as a part of an acid addition salt and a metal complex.

### (1) Plant disease control efficacy

The agricultural or horticultural chemical in the present embodiment exhibits the control efficacy against a wide range of plant diseases. In particular, the agricultural or horticultural chemical exhibits the antimicrobial effect against a wide range of plant pathogenic microorganisms.

### (1-1) Disease

Examples of applicable diseases include the following. Note that, in the parenthesis after each disease name, a major pathogenic microorganism that causes the disease is indicated. Soybean rust (Phakopsora pachyrhizi), soybean rust (Phakopsora meibomiae), soybean brown spot (Septoria glycines), soybean purple stain (Cercospora kikuchii), soybean leaf spot (Alternaria sp.), soybean anthracnose (Colletotrichum truncatum, Colletotrichum glysines), soybean leaf spot (Cercospora sojina), Rhizoctonia root rot of soybean (Rhizoctonia solani), soybean Rhizoctonia rot (Rhizoctonia solani), soybean pod and stem blight (Diaporthe phaseolorum), soybean stem and root rot (Phytophthora sojae), soybean Fusarium head blight (Fusarium avenaceum), soybean Fusarium head blight (Fusarium oxysporum), soybean wilt disease (Verticillium dahliae), soybean powdery mildew (Erysiphe glycines), soybean target spot (Corynespora cassiicola), soybean brown leaf spot (Mycosphaerella sojae), soybean root necrosis (Calonectria ilicicola), soybean charcoal rot (Macrophomina phaseolina), soybean black root rot (Thielaviopsis sp.), soybean ring spot (Ascochyta phaseolorum), soybean anthracnose (Elsinoe glycines), soybean sudden death syndrome (the genus Fusariumu such as Fusarium virguliforme), kidney bean anthracnose (Colletotrichum lindemuthianum), phoma stem canker/stem canker of rapeseed (Leptosphaeria maculans, Leptosphaeria biglobosa), light leaf spot of rapeseed (Pyrenopeziza brassicae), rapeseed clubroot (Plasmodiophora brassicae), Verticillium wilt disease of rapeseed (Verticillium longisporum), Alternaria Blackspot (Alternaria spp.) of rapeseed, rice blast (Pyricularia oryzae), rice leaf spot (Cochliobolus myabeanus), rice leaf blight (Xanthomonas oryzae pv. oryzae), rice sheath blight (Rhizoctonia solani), rice stem rot (Helminthosporium sigmoideum), rice bakanae disease (Fusarium fujikuroi), rice seedling blight (Pythium spp.), rice take-all disease (Gaeumannomyces graminis), barley powdery mildew (Blumeria graminis f. sp. hordei), barley black rust (Puccinia graminis), barley yellow rust (Puccinia striiformis), barley dwarf leaf rust (Puccinia hordei), barley stripe (Pyrenophora graminea), barley scald (Rhynchosporium secalis), barley loose smut (Ustilago nuda), barley net blotch (Pyrenophora teres), barley Fusarium head blight (Fusarium graminearum), barley scab (Microdochium nivale), wheat powdery mildew (Erysiphe graminis f. sp. tritici), wheat leaf rust (Puccinia recondita), wheat yellow rust (Puccinia striiformis), wheat eyespot (Oculimacula yallundae), wheat eyespot (Oculimacula acuformis), wheat Fusarium head blight (Fusarium graminearum), wheat Fusarium head blight (Microdochium nivale), wheat glume blotch (Phaeosphaeria nodorum), wheat leaf blight (Zymoseptoria tritici), wheat pink snow mold (Microdochium nivale), wheat brown snow mold (Pythium iwayamai, Pythium paddicum, Pythium spp.), wheat take-all disease (Gaeumannomyces graminis), wheat glume spot (Epicoccum spp. (Epicoccum nigrum)), wheat yellow spot (Pyrenophora triticirepentis), wheat small-grained sclerotinia rot (Typhula incarnata), wheat small-grained sclerotinia rot (Typhula ishikariensis), wheat sclerotinia snow blight (Sclerotinia borealis), wheat Ramularia leaf spot (Ramularia collo-cygni), grass dollar spot (Sclerotinia homoeocarpa), grass large patch (Rhizoctonia solani), grass brown patch (Rhizoctonia solani), grass anthracnose (Colletotrichum graminicola), gray leaf spot of grass (Pyricularia grisea), necrotic ring spot disease of grass (Ophiosphaerella korrae), red thread of grass (Laetisaria fuciformis), grass rust (Puccinia zoysiae), summer patch disease of grass (Magnaporthe poae), take-all patch of grass (Gaeumannomyces graminis), brown ring patch of grass (Waitea circinata), fairy ring disease of grass (Agaricus spp., Calvatia cyathiformis, Chlorophyllum molybdites, Clitocybe spp., Lepiota spp., Lepista subnuda, Lycoperdon spp., Marasmius oreades, Scleroderma spp., Tricholoma spp., and the like), pink snow mold of grass (Microdochium nivale), grass small-grained sclerotinia rot (Typhula incarnata), grass small-grained sclerotinia rot (Typhula ishikariensis), grass Curvularia leaf blight (Curvularia spp.), grass pseudo-leaf rot (Ceratobasidium spp.), grass take-all disease (Gaeumannomyces sp., Phialophora sp.), corn smut (Ustilago maydis), corn anthracnose (Colletotrichum graminicola), corn brown leaf spot (Kabatiella zeae), corn gray leaf spot (Cercospora zeae-maydis), corn northern leaf blight (Setosphaeria turcica), corn northern leaf spot (Bipolaris zeicola), corn leaf spot (Physoderma maydis), corn rust (Puccinia spp.), corn leaf spot (Bipolaris maydis), corn yellow leaf spot (Phyllosticta maydis), corn Fusarium head blight (Fusarium asiaticum, Fusarium fujikuroi, Fusarium proliferatum, Fusarium concentricum, Fusarium graminearum, Fusarium verticillioides), corn Phaeosphaeria leaf spot (Phaeosphaeria maydis), sugar cane rust (Puccinia spp.), powdery mildew of cucurbits (Sphaerotheca fuliginea), anthracnose of cucurbits (Colletotrichum orbiculare), anthracnose of cucurbits (Glomerella cingulata), downy mildew of cucurbits (Pseudoperonospora cubensis), cucumber phytophthora blight (Phytophthora capsici), Fusarium wilt of cucurbits (Fusarium oxysporum f. sp. cucumerinum), Fusarium wilt of watermelon (Fusarium oxysporum f. sp. niveum), apple powdery mildew (Podosphaera leucotricha), apple scab (Venturia inaequalis), apple monilia leaf blight (Monilinia mali), apple alternaria blotch (Alternaria alternata apple pathotype), apple Valsa canker (Valsa ceratosperma), pear black spot (Alternaria alternata Japanese pear pathotype), pear powdery mildew (Phyllactinia pyri), pear rust (Gymnosporangium asiaticum), pear scab (Venturia nashicola), strawberry powdery mildew (Podosphaera aphanis), stone fruit brown rot (Monilinia fructicola), citrus blue mold (Penicillium italicum), citrus green mold (Penicillium digitatum), grape powdery mildew (Uncinula necator), grape downy mildew (Plasmopara viticola), grape ripe rot (Glomerella cingulata), grape rust (Phakopsora euvitis), banana leaf spot (black Sigatoka leaf disease) (Mycosphaerella fijiensis), banana leaf spot (yellow Sigatoka leaf disease) (Mycosphaerella musicola), tomato powdery mildew (Erysiphe cichoracearum), tomato ring spot (Alternaria solani), tomato leaf mold (Passalora fulva), eggplant powdery mildew (Erysiphe cichoracearum), potato early blight (Alternaria solani), potato anthracnose (Colletotrichum coccodes), potato powdery mildew (Erysiphe cichoracearum), potato powdery mildew (Leveillula taurica), potato blight (Phytophthora infestans), tobacco powdery mildew (Erysiphe cichoracearum), tobacco brown spot (Alternaria longipes), sugar beet brown leaf spot (Cercospora beticola), sugar beet powdery mildew (Erysiphe betae), sugar beet leaf rot (Thanatephorus cucumeris), sugar beet root rot (Thanatephorus cucumeris), sugar beet black root rot (Aphanomyces cochlioides), radish chlorosis (Fusarium oxysporum f. sp. raphani), tea anthracnose (Discula theaesinensis), tea blister blight (Exobasidium vexans), tea cercospora leaf spot (Pseudocercospora ocellata), tea cercospora leaf spot (Cercospora chaae), tea leaf blight (Pestalotiopsis longiseta), tea leaf blight (Pestalotiopsis theae), tea net blister blight (Exobasidium reticulatum), cotton black spot (Alternaria spp.), cotton anthracnose (Glomerella spp.), cotton ring spot (Ascochyta gossypii), cotton rust (Puccinia spp.), cotton rust (Phakopsora gossypii), Cercospora blight and leaf spot of cotton (Cercospora spp.), Diplopia boll rot of cotton (Diplopia spp.), Hardlock of cotton (Fusarium spp.), Phoma blight of cotton (Phoma spp.), Stemphyllium leaf spot of cotton (Stemphylium spp.), peanut leaf spot (Cercosporidium personatum), peanut brown leaf spot (Cercospora arachidicola), peanut stem rot (Sclerotium rolfsii), peanut rust (Puccinia arachidis), gray mold that affects various crops (Botrytis cinerea), diseases of pythium that affects various crops (Pythium spp.), and sclerotinia rot that affects various crops (Sclerotinia sclerotiorum). Examples also include seed-borne diseases or early-growth diseases of various plants caused by pathogenic microorganisms such as the genus Aspergillus, the genus Cochliobolus, the genus Corticium, the genus Diplodia, the genus Penicillium, the genus Fusarium, the genus Gibberella, the genus Mucor, the genus Phoma, the genus Phomopsis, the genus Pyrenophora, the genus Pythium, the genus Rhizoctonia, the genus Rhizopus, the genus Thielaviopsis, the genus Tilletia, the genus Trichoderma, and the genus Ustilago.

The agricultural or horticultural chemical according to the present embodiment can be used as a fungicide. Furthermore, the agricultural or horticultural chemical according to the present embodiment exhibits a particularly excellent control efficacy against diseases of wheat leaf blight, wheat leaf rust, and wheat powdery mildew, among the diseases described above. Accordingly, the agricultural or horticultural chemical is advantageously used for disease control in wheat, but is not limited to such application.

### (1-2) Crop

The agricultural or horticultural chemical of the present embodiment can be used for all plants, and examples of applicable plants include the following; Poaceae such as rice, wheat, barley, rye, oats, triticale, corn, sorghum, sugar cane, turf, bentgrass, bermudagrass, fescue, and ryegrass; legumes such as soybean, peanut, kidney bean, peas, adzuki beans, and alfalfa; Convolvulaceae such as sweet potatoes; Solanaceae such as capsicum, pepper, tomato, eggplant, potato, and tobacco; Polygonaceae such as buckwheat; Asteraceae such as sunflower; Araliaceae such as ginseng; Brassicaceae such as rapeseed, Chinese cabbage, turnip, cabbage, and Japanese radish; Chenopodiaceae such as sugar beet; Malvaceae such as cotton; Rubiaceae such as coffee tree; Sterculiaceae such as cacao; Theaceae such as tea; Cucurbitaceae such as watermelon, melon, cucumber, and pumpkin; Liliaceae such as onion, leek, and garlic; Rosaceae such as strawberries, apples, almonds, apricots, Japanese apricots, cherry, plums, peaches, and pears; Apiaceae such as carrots; Araceae such as taro; Anacardiaceae such as mango; Bromeliaceae such as pineapples; Caricaceae such as papayas; Ebenaceae such as persimmons; Ericaceae such as blueberries, Juglandaceae such as pecans; Musaceae such as bananas; Oleaceae such as olives; Palmae such as coconut, and date; Rutaceae such as mandarin orange, orange, grapefruit, and lemon; Vitaceae such as grapes; flowers and ornamental plants, trees other than fruit trees; and other ornamental plants. Furthermore, examples include wild plants, cultivars, plants and cultivars bred by known hybridizing or protoplast fusion, and genetically recombinant plants and cultivars obtained by gene manipulation. Examples of genetically recombined plants and cultivars include herbicide-tolerant crops, pest-resistant crops in which an insecticidal protein-producing gene has been recombined, pathogen-resistant crops in which a pathogen resistance derivative-producing gene has been recombined, taste-improved crops, yield-improved crops, preservation-improved crops, and yield-improved crops. Examples of genetically recombined cultivar that has been approved in each country include those stored in the database of the International Service for the Acquisition of Agri-biotech Applications (ISAAA). Specific examples include ones including registered trademark such as AgriSure, AgriSure 3000 GT, AgriSure 3122 E-Z Refuge, AgriSure 3122 Refuge Renew, AgriSure Artesian 3030 A, AgriSure Artesian 3011 A, AgriSure Duracade, AgriSure Duracade 5222 E-Z Refuge, AgriSure GT, AgriSure GT/CB/LL, AgriSure RW, AgriSure Viptera 3110, AgriSure Viptera 3111, AgriSure Viptera 3220 E-Z Refuge, AgriSure Viptera 3220 Refuge Renew, BiteGard, Bollgard, Bollgard II, Bollgard II, Bollgard II/Roundup Ready, Bollgard 3 XtendFlex Cotton, Bollgard Cotton, Bollgard/Roundup Ready Cotton, B.t., B.t/BXN Cotton, B.t. Maize, BtXtra, BXN, BXN Canola, BXN Cotton, Clearfield, DroughtGard, Enlist, Enlist Cotton, Enlist WideStrike 3 Cotton, Genuity, Genuity Bollgard II XtendFlex, Genuity Intacta RR2 Pro, Genuity SmartStax, Genuity SmartStax RIB Complete, Genuity VT Double Pro, Genuity VT Double Pro RIB Complete, Genuity VT Triple Pro, Genuity VT Triple Pro RIB Complete, GlyTol, GlyTol Cotton, Herculex, Herculex 1, Herculex RW, Herculex XTRA, IMI, IMI Canola, InVigor, KnockOut, Liberty Link, Liberty Link Conola, Liberty Link cotton, NatureGard, Newleaf, Nucotn, Optimum, Optimum AcreMax, Optimum AcreMax I, Optimum AcreMax-R, Optimum AcreMax RW, Optimum AcreMax RW-R, Optimum AcreMax Xtra-R, Optimum AcreMax Xtreme-R, Optimum AcreMax Xtreme, Optimum Intrasect, Optimum Intrasect Xtra, Optimum Intrasect Xtreme, Optimum Leptra, Optimum TRIsect, Poast Compatible, Powercore, Powercore Corn, Powercore Corn Refuge Advanced, Protecta, Roundup Ready, Roundup Ready 2, Roundup Ready Conola, Roundup Ready Cotton, Roundup Ready Xtend, Roundup Ready/YieldGard, RR Flex/Bollgard II, SCS, SmartStax, SmartStax Refuge Advanced, StarLink, Twinlink, VipCot, VipCot Cotton, WideStrike, WideStrike 3, YieldGard, YieldGard Corn Borner, YieldGard Rootworm, YieldGard Plus, and YieldGard VT Triple.

### (2) Formulation

The agricultural or horticultural chemical is used by mixing the pyrazole derivative (I) as an active ingredient with a carrier, a surfactant, and other formulation aids to be used in various forms such as dustable powders, granules, dust-granule mixtures, wettable powders, water soluble powders, emulsifiable concentrates, soluble concentrates, oil or oil miscible liquids, aerosols, microcapsules, pastes, coating agents, smoke agents, fumigants, and ultra-low volume formulations. These formulations are prepared so that pyrazole derivative (I) is contained as an active ingredient in an amount of 0.1 to 95 wt.%, preferably 0.5 to 90 wt.%, and more preferably 1 to 80 wt.%.

Examples of the carrier to be used as a formulation aid include solid carriers and liquid carriers. Examples of the solid carrier include those used as powder carriers and granular carriers, such as minerals such as clay, talc, diatomaceous earth, zeolite, montmorillonite, bentonite, kaolinite, kaolin, pyrophyllite, agalmatolite, acid clay, activated clay, attapulgite, attapulgite clay, limestone, calcite, marble, vermiculite, perlite, pumice, silica stone, silica sand, sericite and porcelain stone; synthetic organic substances such as urea; salts such as calcium carbonate, sodium carbonate, magnesium carbonate, sodium sulfate, ammonium sulfate, potassium chloride, slaked lime, and sodium bicarbonate; synthetic inorganic substances such as amorphous silica (e.g., precipitated silica and fumed silica) and titanium dioxide; plant carriers such as wood flour, corn stalk (cob), walnut shell (nut shell), fruit stone, rice hull, coconut shell, sawdust, bran, soy flour, powdered cellulose, starch, dextrin, and sugars (e.g., lactose and sucrose); and various polymeric carriers such as crosslinked lignin, cation gel, gelatin gelated by heat or a polyvalent metal salt, water-soluble polymer gel (e.g., agar), chlorinated polyethylene, chlorinated polypropylene, polyvinyl acetate, polyvinyl chloride, ethylene-vinyl acetate copolymers, and urea-aldehyde resins.

Examples of the liquid carrier include
aliphatic solvents such as paraffins (normal paraffin, iso-paraffin, naphthene); aromatic solvents such as xylene, alkylbenzene, alkylnaphthalene, and solvent naphtha; mixed solvents such as kerosene; machine oils such as refined high-boiling point aliphatic hydrocarbons; alcohols such as methanol, ethanol, isopropanol, butanol, and cyclohexanol; polyhydric alcohols such as ethylene glycol, diethylene glycol, propylene glycol, dipropylene glycol, hexylene glycol, polyethylene glycol, and polypropylene glycol; polyhydric alcohol derivatives such as propylene glycol ether; ketones such as acetone, acetophenone, cyclohexanone, methylcyclohexanone, and γ-butyrolactone; esters such as fatty acid methyl ester (coconut oil fatty acid methyl ester), ethylhexyl lactate, propylene carbonate, dibasic acid methyl ester (dimethyl succinate, dimethyl glutamate, and dimethyl adipate); nitrogen-containing solvents such as N-alkylpyrrolidones and acetonitrile; sulfur-containing solvents such as dimethyl sulfoxide; oils and fats such as coconut oil, soybean oil, and rapeseed oil; amide-based solvents such as dimethylformamide, N,N-dimethyloctanamide, N,N-dimethyldecanamide, methyl 5-(dimethylamino)-2-methyl-5-oxo-valerate, and N-acylmorpholine-based solvents (CAS No. 887947-29-7 and the like); and water.

Examples of the surfactant used as the formulation aid include nonionic surfactants, anionic surfactants, cationic surfactants, amphoteric surfactants, silicone surfactants, fluorosurfactants, and biosurfactants. Examples of the nonionic surfactants include
sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkylphenyl ether, polyoxyethylene dialkylphenyl ether, polyoxyethylene alkyl phenyl ether formaldehyde condensate, polyoxyethylene/polyoxypropylene block polymer, alkyl polyoxyethylene/polyoxypropylene block polymer ether, alkyl phenyl polyoxyethylene/polyoxypropylene block polymer ether, polyoxyethylene alkylamine, polyoxyethylene fatty acid amide, polyoxyethylene bisphenyl ether, polyoxyethylene benzylphenyl (or phenylphenyl) ether, polyoxyethylene styrylphenyl (or phenylphenyl) ether, polyoxyethylene castor oil, polyoxyethylene hydrogenated castor oil, and alkyl glycosides.

Examples of the anionic surfactants include
sulfates such as alkyl sulfate, polyoxyethylene alkyl ether sulfate, polyoxyethylene alkylphenyl ether sulfate, polyoxyethylene benzyl (or styryl) phenyl (or phenylphenyl) ether sulfate, polyoxyethylene/polyoxypropylene block polymer sulfate; sulfonates such as paraffin (alkane) sulfonate, α-olefin sulfonate, dialkyl sulfosuccinate, alkylbenzene sulfonate, mono- or dialkyl naphthalene sulfonate, naphthalene sulfonate-formaldehyde condensate, alkyl diphenyl ether disulfonate, lignin sulfonate, polyoxyethylene alkylphenyl ether sulfonate, and polyoxyethylene alkyl ether sulfosuccinate half ester; carboxylates such as fatty acids, resin acids, polycarboxylic acids, alkyl ether carboxylates, alkenyl succinic acids, N-acyl amino acid, and naphthenic acid; and phosphates such as polyoxyethylene alkyl ether phosphate, polyoxyethylene mono- or dialkyl phenyl ether phosphate, polyoxyethylene benzyl (or styryl) phenyl (or phenylphenyl) ether phosphate, polyoxyethylene/polyoxypropylene block polymer phosphates, and alkyl phosphates.

Examples of the cationic surfactants include
salts of amines such as alkylamines and alkyl pentamethyl propylene diamine; salts of ammoniums such as alkyltrimethylammonium, methyl polyoxyethylene alkylammonium, alkyl pyridinium, mono- or di-alkyl methylated ammonium, alkyl pentamethylpropylenediamine, alkyl dimethyl benzalkonium, and benzethonium (octylphenoxyethoxy ethyl dimethyl benzylammonium).

Examples of the amphoteric surfactants include dialkyldiamino ethyl betaine, alkyldimethyl benzyl betaine, and lecithin (e.g., phosphatidylcholine and phosphatidylethanolamine).

Examples of the silicone surfactants include trisiloxane ethoxylate.

Examples of the fluorosurfactants include perfluoroalkyl carboxylates, perfluoroalkyl sulfonates, and perfluoroalkyl trimethylammonium salts.

Examples of the biosurfactants include
glycolipids, such as sophorolipids, rhamnolipids, trehalose lipids, mannosyl alditol lipids, cellobiose lipids, glucose lipids, and oligosaccharide fatty acid esters; fatty acids, such as spiculisporic acids, corynomycolic acids, and agaritinic acids; acylpeptides, such as surfactins, serrawettins, viscosins, lichenysins, and arthrofactins; and macromolecules, such as emulsans and alasans.

Examples of the other formulation aids include
inorganic salts used as pH regulators (such as sodium or potassium); sodium chloride used as water-soluble salts; xanthan gum, guar gum, carboxymethyl cellulose, polyvinylpyrrolidone, carboxy vinyl polymers, acrylic polymers, polyvinyl alcohols, starch derivatives, water-soluble polymers (such as polysaccharides), alginic acid and salts thereof, which are used as thickeners; metal stearates, sodium tripolyphosphate, and sodium hexametaphosphate, which are used as disintegration dispersing agents; benzoic acid and salts thereof, sorbic acid and salts thereof, propionic acid and salts thereof, p-hydroxybenzoic acid, methyl p-hydroxybenzoate, and 1,2-benzothiazolin-3-one, which are used as preservatives; sodium polyphosphate, sodium polyacrylate, sodium ligninsulfonate, sodium citrate, sodium gluconate/glucoheptonate, ethylenediaminetetraacetic acid and disodium salts thereof and ammonium salts thereof, which are used as supplementary agents; pigments and dyes used as colorants, fluorine-based antifoaming agents, silicone-based antifoaming agents, and ethylene oxide/propylene oxide copolymers, which are used as antifoaming agents; phenol-based antioxidants, amine-based antioxidants, sulfur-based antioxidants, and phosphoric acid-based antioxidants, which are used as antioxidants; salicylic acid-based UV absorbers and benzophenone-based UV absorbers, which are used as UV absorbers; calcium oxide and magnesium oxide, which are used as desiccants; and other spreaders and safeners.

Some formulations are used as they are and some are diluted with a diluent such as water to a predetermined concentration before use. The concentration of pyrazole derivative (I) when diluted before use is preferably in the range of 0.001 to 1.0%.

The amount of pyrazole derivative (I) used is from 20 to 5000 g, and more preferably from 50 to 2000 g per 1 ha of agricultural or horticultural area such as fields, rice fields, orchards, and greenhouses. Since these concentration and quantity used differ depending on the form of the agent, time of use, usage method, usage location, target crops, and the like, they may be increased or decreased as appropriate and are not limited to the ranges stated above.

### (3) Other active ingredients

The agricultural or horticultural chemical in the present embodiment may be used in combination with other known active ingredients to enhance the performance as an agricultural or horticultural chemical. Examples of the other known active ingredients include known active ingredients contained in fungicides, insecticides, miticides, nematicides, herbicides, and plant growth regulators.

### (3-1) Active ingredients for fungicidal use

Examples of the active ingredients suitable for use in fungicides include nucleic acid synthesis and metabolism inhibitors, fungicides acting on cytoskeleton and motor proteins, respiratory inhibitors, amino acid/protein biosynthesis inhibitors, signal transduction inhibitors, lipid biosynthesis or transport/cell membrane structure or function inhibitors, cell membrane sterol biosynthesis inhibitors, cell wall biosynthesis inhibitors, melanin biosynthesis inhibitors, host plant resistance inducers, multi-site fungicides, and biopesticides/agrochemicals of biological origin having a plurality of action mechanisms.

Specifically, examples of the nucleic acid synthesis and metabolism inhibitors include
benalaxyl, benalaxyl-M or kiralaxyl, furalaxyl, metalaxyl, metalaxyl-M or mefenoxam, ofurace, oxadixyl, bupirimate, dimethirimol, ethirimol, hydroxyisoxazole, octhilinone, and oxolinic acid.

Examples of the fungicides acting on cytoskeleton and motor proteins include
benomyl, carbendazim, fuberidazole, thiabendazole, thiophanate, thiophanate-methyl, diethofencarb, ethaboxam, pencycuron, zoxamide, fluopicolide, fluopimomide, phenamacril, metrafenone, and pyriofenone.

Examples of the respiration inhibitors include
diflumetorim, fenazaquin, tolfenpyrad, benodanil, benzovindiflupyr, bixafen, boscalid, carboxin, fenfuram, flubeneteram, fluindapyr, fluopyram, flutolanil, fluxapyroxad, furametpyr, inpyrfluxam, isofetamid, isoflucypram, isopyrazam, mepronil, oxycarboxin, penflufen, penthiopyrad, pydiflumetofen, pyrapropoyne, pyraziflumid, sedaxane, thifluzamide, azoxystrobin, coumoxystrobin, dimoxystrobin, enestrobin, enoxastrobin, famoxadone, fenamidone, fenaminstrobin, flufenoxystrobin, fluoxastrobin, kresoxim-methyl, mandestrobin, metominostrobin, metyltetraprole, orysastrobin, picoxystrobin, pyraclostrobin, pyrametostrobin, pyraoxystrobin, pyribencarb, triclopyricarb, trifloxystrobin, amisulbrom, cyazofamid, fenpicoxamid, florylpicoxamid, metarylpicoxamid, binapacryl, DPC (dinocap), fluazinam, meptyldinocap, triphenyltin acetate, triphenyltin chloride, triphenyltin hydroxide, silthiofam, and ametoctradin.

Examples of the amino acid/protein biosynthesis inhibitors include cyprodinil, mepanipyrim, pyrimethanil, blasticidin-S, kasugamycin, streptomycin, and oxytetracycline.

Examples of the signal transduction inhibitors include
proquinazid, quinoxyfen, fludioxonil, chlozolinate, dimethachlone, fenpiclonil, iprodione, procymidone, and vinclozolin.

Examples of the lipid biosynthesis or transport/cell membrane structure or function inhibitors include edifenphos (EDDP), iprobenfos (IBP), isoprothiolane, pyrazophos, biphenyl, chloroneb, dicloran (CNA), etridiazole, quintozene (PCNB), tecnazene (TCNB), tolclofos-methyl, iodocarb, propamocarb, prothiocarb, extracts of Melaleuca alternifolia (tea tree), plant oil mixtures (eugenol, geraniol, thymol), natamycin (pimaricin), fluoxapiprolin, and oxathiapiprolin.

Examples of the cell membrane sterol biosynthesis inhibitors include azaconazole, bitertanol, bromuconazole, cyproconazole, difenoconazole, diniconazole, epoxiconazole, etaconazole, fenbuconazole, fluoxytioconazole, fluquinconazole, flusilazole, flutriafol, hexaconazole, imazalil, imibenconazole, ipconazole, ipfentrifluconazole, mefentrifluconazole, metconazole, myclobutanil, oxpoconazole, pefurazoate, penconazole, prochloraz, propiconazole, prothioconazole, simeconazole, tebuconazole, tetraconazole, triadimefon, triadimenol, triflumizole, triticonazole, fenarimol, nuarimol, pyrifenox, pyrisoxazole, triforine, methyl (2RS)-2-[2-chloro-4-(4-chlorophenoxy)phenyl]-2-hydroxy-3-(1H-1,2,4-triazol-1-yl)propanoate, 1-((1H-1,2,4-triazol-1-yl)methyl)-5-(4-chlorobenzyl)-2-(chloromethyl)-2-methylcyclopentan-1-ol, methyl2-((1H-1,2,4-triazol-1-yl)methyl)-3-(4-chlorobenzyl)-2-hydroxy-1-methylcyclopentane-1-carboxylate, aldimorph, dodemorph, fenpropimorph, tridemorph, fenpropidin, piperalin, spiroxamine, fenhexamid, fenpyrazamine, pyributicarb, naftifine, and terbinafine.

Examples of the cell wall biosynthesis inhibitors include
polyoxin, benthiavalicarb (benthiavalicarb-isopropyl), dimethomorph, flumorph, iprovalicarb, mandipropamid, pyrimorph, and valifenalate.

Examples of the melanin biosynthesis inhibitors include phthalide, pyroquilon, tricyclazole, carpropamid, diclocymet, fenoxanil, and tolprocarb.

Examples of the host plant resistance inducers include
acibenzolar-S-methyl, probenazole (also having antifungal activity), tiadinil, isotianil, laminarin, Reynoutria sachalinensis extracts, Bacillus mycoides isolate J, cell wall of Saccharomyces cerevisiae strain LAS117, fosetyl (Fosetyl-aluminum, Fosetyl potassium, Fosetyl sodium), phosphoric acid, phosphates, and dichlobentiazox.

Examples of the multi-site fungicides include
ferbam, manzeb, maneb, metiram, propineb, thiuram, zinc thiazole, zineb, ziram, amobam, anilazine, dithianon, dichlofluanid, tolylfluanid, guazatine, iminoctadine acetate/iminoctadine albesilate (iminoctadine), copper or various copper salts (such as basic copper chloride, copper(II) hydroxide, basic copper sulfate, copper sulfate, organocopper (oxine-copper), copper nonylphenolsulfonate, DBEDC), sulfur, captan, captafol, folpet, chlorothalonil (TPN), quinoxaline system (chinomethionate), fluoroimide, and metasulfocarb.

Examples of biopesticides/agrochemicals of biological origin having a plurality of action mechanisms include
chloroinconazide, seboctylamine, flumetylsulforim, flufenoxadiazam, cyflufenamid, cymoxanil, diclomezine, dipymetitrone, dodine, fenitropan, ferimzone, flusulfamide, flutianil, harpin, inorganic salts (hydrogencarbonates (sodium hydrogencarbonate, potassium hydrogencarbonate), potassium carbonate), ipflufenoquin, KINOPROL, materials of biological origin, machine oils, organic oils, picarbutrazox, pyridachlometyl, quinofumelin, tebufloquin, tecloftalam (bactericides), triazoxide, validamycin, aminopyrifen, and shiitake mycelium extracts.

Examples of other compounds for use in fungicides include
Bacillus subtilis strain AFS032321, Bacillus amyloliquefaciens strain QST713, Bacillus amyloliquefaciens strain FZB24, Bacillus amyloliquefaciens strain MBI600, Bacillus amyloliquefaciens strain D747, Bacillus amyloliquefaciens strain F727, Clonostachys rosea strain CR-7, Gliocladium catenulatum strain J1446, Pseudomonas chlororaphis strain AFS009, Streptomyces griseoviridis strain K61, Streptomyces lydicus strain WYEC108, Trichoderma atroviride strain I-1237, Trichoderma atroviride strain LU132, Trichoderma atroviride strain SC1, Trichoderma asperellum strain T34, extracts from Swinglea glutinosa, and extracts from the cotyledons of Lupine seedlings.

### (3-2) Active Ingredients for Use in Insecticides, Miticides, and Nematicides

Examples of the active ingredients suitable for use in insecticides, miticides, and nematicides include acetylcholinesterase (AChE) inhibitors, GABA-gated chloride ion channel blockers, sodium channel modulators, nicotinic acetylcholine receptor (nAChR) competitive modulators, nicotinic acetylcholine receptor (nAChR) allosteric modulators, glutamate-gated chloride ion channel (GluCl) allosteric modulators, juvenile hormone mimics, other non-specific (multi-site) inhibitors, chordotonal organ TRPV channel modulators, mite growth inhibitors affecting CHS1, microorganism-derived insect midgut inner membrane disrupting agents, mitochondrial ATP synthase inhibitors, oxidative phosphorylation uncouplers via disruption of proton gradient, nicotinic acetylcholine receptor (nAChR) channel blockers, chitin biosynthesis inhibitors affecting CHS1, chitin biosynthesis inhibitors (type 1), molting inhibitor (insects of the order Diptera), molting hormone (ecdysone) receptor agonists, octopamine receptor agonists, mitochondrial electron transport complex III inhibitors, mitochondrial electron transport complex I inhibitors (METI), voltage-dependent sodium channel blockers, acetyl-CoA carboxylase inhibitors, mitochondrial electron transport complex IV inhibitors, mitochondrial electron transport complex II inhibitors, ryanodine receptor modulators, chordotonal organ modulators, GABA-gated chloride ion channel allosteric modulators, and baculoviruses.

Examples of the acetylcholinesterase (AChE) inhibitors include alanycarb, aldicarb, bendiocarb, benfuracarb, butocarboxim, butoxycarboxim, carbaryl (NAC), carbofuran, carbosulfan, ethiofencarb, fenobucarb (BPMC), fenothiocarb, formetanate, furathiocarb, isoprocarb (MIPC), methiocarb, methomyl, metolcarb (MTMC), oxamyl, pirimicarb, propoxur (PHC), thiodicarb, thiofanox, triazamate, trimethacarb, XMC, xylylcarb (MPMC), acephate, azamethiphos, azinphos-ethyl, azinphos-methyl, cadusafos, chlorethoxyfos, chlorfenvinphos (CVP), chlormephos, chlorpyrifos, chlorpyrifos-methyl, coumaphos, cyanophos (CYAP), demeton-S-methyl, diazinon, dichlorvos (DDVP), dicrotophos, dimethoate, dimethylvinphos, ethylthiomethon (disulfoton), EPN, ethion, ethoprophos, famphur, fenamiphos, fenitrothion (MEP), fenthion (MPP), fosthiazate, heptenophos, imicyafos, isofenphos, isopropyl O-(methoxyaminothiophosphoryl)salicylate, isoxathion, malathon (malathion), mecarbam, methamidophos, methidathion (DMTP), mevinphos, monocrotophos, naled (BRP), omethoate, oxydemeton-methyl, parathion, methyl parathion (parathion-methyl), phenthoate (PAP), phorate, phosalone, phosmet (PMP), phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos (CVMP), thiometon, triazophos, trichlorfon (DEP), and vamidothion.

Examples of the GABA-gated chloride ion channel blockers include chlordane, benzoepin (endosulfan), dienochlor, ethiprole, fipronil, pyriprole, and nicofluprole.

Examples of the sodium channel modulators include
acrinathrin, allethrin (allethrin, d-cis-trans-, d-trans-isomers), bifenthrin, bioallethrin (bioallethrin, S-cyclopentenyl-isomers), bioresmethrin, chloroprallethrin, chlorfenson, cycloprothrin, cyfluthrin (cyfluthrin, β-isomers), cyhalothrin (cyhalothrin, λ-, γ-isomers), cypermethrin (cypermethrin, α-, β-, θ-, ζ-isomers), cyphenothrin [(1R)-trans-isomers], deltamethrin, dimefluthrin, empenthrin [(EZ)-(1R)-isomers], esfenvalerate, etofenprox, fenpropathrin, fenvalerate, flubrocythrinate, flucythrinate, flumethrin, fluvalinate (τ-fluvalinate), halfenprox, imiprothrin, kadethrin, metofluthrin, momfluorothrin, epsilon-metofluthrin, epsilon-momfluorothrin, permethrin, phenothrin [(1R)-trans-isomer], prallethrin, profluthrin, pyrethrin, resmethrin, silafluofen, tefluthrin, phthalthrin (tetramethrin), tetramethrin [(1R)-isomers], tralomethrin, transfluthrin, DDT, methoxychlor, aldrin, dieldrin, and lindane (lindene).

Examples of the nicotinic acetylcholine receptor (nAChR) competitive modulators include
acetamiprid, clothianidin, dinotefuran, imidacloprid, nitenpyram, thiacloprid, thiamethoxam, nicotine sulfate (nicotine), sulfoxaflor, flupyradifurone, triflumezopyrim, dicloromezotiaz, and fenmezoditiaz.

Examples of the nicotinic acetylcholine receptor (nAChR) allosteric modulators include spinetoram, spinosad, and flupyrimin.

Examples of the glutamate-gated chloride ion channel (GluCl) allosteric modulators include
abamectin, emamectin benzoate, lepimectin, and milbemectin.

Examples of the juvenile hormone mimics include
hydroprene, kinoprene, methoprene, fenoxycarb, and pyriproxyfen.

Examples of other non-specific (multi-site) inhibitors include
methyl bromide, other alkyl halides, chloropicrin, sodium aluminum fluoride, sulfuryl fluoride, borax, boric acid, disodium octaborate, sodium metaborate, tartar emetic, dazomet, carbam (metam-ammonium), metam-sodium (carbam sodium), and methyl isothiocyanate.

Examples of the chordotonal organ TRPV channel modulators include pymetrozine, pyrifluquinazon, and afidopyropen.

Examples of the mite growth inhibitors affecting CHS1 include clofentezine, diflovidazin, hexythiazox, and etoxazole.

Examples of the microbial disruptors of insect midgut membranes include
B.t. subsp. israelensis, B.t. subsp. aizawai, B.t. subsp. kurstaki, B.t. subsp. tenebrionis, proteins contained in B.t. crops: Cry1Ab, Cry1Ac, Cry1Fa, Cry1A.105, Cry2Ab, Vip3A, mCry3A, Cry3Ab, Cry34Ab1/Cry35Ab1, and Bacillus sphaericus.

Examples of the mitochondrial ATP synthase inhibitors include diafenthiuron, azocyclotin, tricyclohexyltin hydroxide (cyhexatin), fenbutatin oxide, propargite (BPPS), and tetradifon.

Examples of the oxidative phosphorylation uncouplers via disruption of proton gradient include chlorfenapyr, DNOC, and sulfluramid.

Examples of the nicotinic acetylcholine receptor (nAChR) channel blockers include
bensultap, cartap hydrochloride, thiocyclam, thiosultap sodium, and monosultap.

Examples of the chitin biosynthesis inhibitors affecting CHS1 include cartap hydrochloride, thiocyclam, thiosultap sodium, monosultap, bistrifluron, chlorfluazuron, diflubenzuron, flucycloxuron, flufenoxuron, hexaflumuron, lufenuron, novaluron, noviflumuron, teflubenzuron, and triflumuron.

Examples of the chitin biosynthesis inhibitors (type 1) include buprofezin.

Examples of the molting inhibitors (insects of the order Diptera) include cyromazine.

Examples of the molting hormone (ecdysone) receptor agonists include chromafenozide, halofenozide, methoxyfenozide, and tebufenozide.

Examples of the octopamine receptor agonists include amitraz.

Examples of the mitochondrial complex III electron transport inhibitors include
hydramethylnon, acequinocyl, fluacrypyrim, bifenazate, and flupyroxystrobin.

Examples of the mitochondrial electron transport complex I inhibitors (METI) include
fenazaquin, fenpyroximate, pyridaben, pyrimidifen, tebufenpyrad, tolfenpyrad, and derris (rotenone).

Examples of the voltage-dependent sodium channel blockers include indoxacarb and metaflumizone.

Examples of the acetyl-CoA carboxylase inhibitors include
spirodiclofen, spiromesifen, spiropidion, spirotetramat, spidoxamat, and spirobudifen.

Examples of the mitochondrial electron transport complex IV inhibitors include
aluminum phosphide, calcium phosphide, hydrogen phosphide, zinc phosphide, prussic acid (calcium cyanide, sodium cyanide), and potassium cyanide.

Examples of the mitochondrial electron transport complex II inhibitors include cyenopyrafen, cyetpyrafen, cyflumetofen, pyflubumide, and cyclobutrifluram #E-25.

Examples of the ryanodine receptor modulators include
chlorantraniliprole, cyantraniliprole, cyclaniliprole, flubendiamide, tetraniliprole, fluchlordiniliprole, tetrachlorantraniliprole, cyhalodiamide, cyproflanilide, and thiorantraniliprole.

Examples of the chordotonal organ modulators include flonicamid.

Examples of the GABA-gated chloride ion channel allosteric modulators include
broflanilide, fluxametamide, and isocycloseram.

Examples of the baculoviruses include
codling moth (Cydia pomonella GV), false codling moth (Thaumatotibia leucotreta GV), velvetbean caterpillar (Anticarsis gemmatalis MNPV), and cotton bollworm (Helicoverpa armigera NPV).

Examples of the nicotinic acetylcholine receptor (nAChR) allosteric modulators include
GS-omega/kappa HXTX-Hv1a peptide.

Examples of RNAi include ledprona.

Examples of other insecticides, miticides, and nematicides include azadirachtin, benzomate (benzoximate), phenisobromolate (bromopropylate), quinoxaline system (chinomethionate), kelthane (dicofol), lime sulfur, mancozeb, pyridalyl, sulfur, acynonapyr, amidoflumet, benzpyrimoxan, fluazaindolizine, fluensulfone, fluhexafon, flupentiofenox, flometoquin, metaldehyde, tyclopyrazoflor, dimpropyridaz, trifluenfuronate, indazapyroxamet, sulfiflumin, Burkholderia spp., Wolbachia pipientis (Zap), Chenopodium ambrosioides near ambrosioides extracts, fatty acid monoesters with glycerol or propanediol, neem oil, machine oil, rapeseed oil, blended oils, starch, reduced starch hydrolysates, sodium oleate, ferric phosphate, nemadectin, Beauveria bassiana strains, Metarhizium anisopliae strain (F52), Paecilomyces fumosoroseus Apopka strain (97), diatomaceous earth, dichlorodiisopropyl ether (DCIP), 1,3-dichloropropene (D-D), levamisole hydrochloride, morantel tartrate, and tioxazafen.

### (3-5) Active ingredients for plant growth regulator use

Examples of the optimal active ingredients for use in the plant growth regulator include
aminoethoxyvinylglycine, chlormequat, chlorpropham, cyclanilide, dikegulac, daminozide, ethephon, flurprimidol, flumetralin, forchlorfenuron, gibberellin, maleic hydrazide salt, mepiquat chloride, methylcyclopropene, benzylaminopurine, paclobutrazol, prohexadione, thidiazuron, tribufos(tributylphosphorotrithioate), trinexapac-ethyl, uniconazole, sodium 1-naphthaleneacetate, 1-naphthylacetamide, 1-methylcyclopropene, 4-chlorophenoxyacetic acid (4-CPA), ethyl (4-chloro-2-methylphenoxy)butyrate (MCPB), isoprothiolane, itaconic acid, indole butyric acid, ethychlozate, calcium formate, chlormequat, choline, cyanamide, dichlorprop, decyl alcohol, sorbitan trioleate, nicosulfuron, pyraflufen-ethyl, butralin, prohydrojasmon, pendimethalin, and anisiflupurin.

### (4) Method for controlling plant diseases

The agricultural or horticultural chemical in the present embodiment may be used, for example, in cultivated lands such as fields, paddy fields, lawns, and orchards or non-cultivated lands. In addition, the agricultural or horticultural chemical in the present embodiment can be applied not only by foliage treatment such as spraying foliage, but also by non-foliage treatment such as seed treatment including treatment of bulbs and tubers, soil-drenching treatment, and water surface treatment. Therefore, the method for controlling plant diseases of the present embodiment includes a step of performing foliage treatment or non-foliage treatment using the agricultural or horticultural chemical agent described above. When non-foliage treatment is performed, the amount of labor required can be reduced in comparison to when foliage treatment is performed.

In seed treatment, the chemical is applied to the seeds by mixing and stirring a wettable powder and a dustable powder with the seeds, or by dipping the seeds in a diluted wettable powder. The seed treatment also includes seed coating treatment. The amount of active ingredients used in the case of seed treatment is, for example, from 0.01 to 10000 g, and preferably from 0.1 to 1000 g per 100 kg of seeds. Seeds treated with agricultural or horticultural chemicals can be used in the same way as common seeds.

In the case of application by soil-drenching treatment, a planting hole or the vicinity thereof may be treated with granules or the like at the time of the transplantation of seedling or the like, the soil around seeds or a plant may be treated with granules, a wettable powder, or the like. The amount of active ingredients used in the case of soil-drenching treatment is, for example, from 0.01 to 10,000 g and preferably from 0.1 to 1,000 g per 1 m² of agricultural or horticultural area.

In the case of application by water surface treatment, the water surface of a paddy field may be treated with granules or the like. The amount of active ingredients used in the case of water surface treatment is, for example, from 0.1 to 10000 g and preferably from 1 to 1000 g per 10 a of the paddy field.

The amount of active ingredients used for foliar spraying is, for example, from 20 to 5,000 g, preferably from 50 to 2,000 g per hectare of agricultural or horticultural area such as fields, rice paddies, orchards, and greenhouses.

These concentrations and amounts can be increased or decreased without regard to the above ranges, as they depend on the formulation, time of use, method of use, location of use, and target crops.

### 5. Industrial Material Protectant

### (1) Industrial material protection effect

The pyrazole derivative (I) exhibits an excellent effect in protecting materials from a wide variety of harmful microorganisms that erode industrial materials, and thus can be also used for industrial material protectants. Examples of such microorganisms include the microorganisms listed below.

Examples thereof include aspergillus (Aspergillus sp.), trichoderma (Trichoderma sp.), penicillium (Penicillium sp.), geotrichum (Geotrichum sp.), chaetomium (Chaetomium sp.), cadophora (Cadophora sp.), ceratostomella (Ceratostomella sp.), cladosporium (Cladosporium sp.), corticium (Corticium sp.), lentinus (Lentinus sp.), lenzites (Lenzites sp.), phoma (Phoma sp.), polysticus (Polysticus sp.), pullularia (Pullularia sp.), stereum (Stereum sp.), trichosporium (Trichosporium sp.), aerobacter (Aerobacter sp.), bacillus (Bacillus sp.), desulfovibrio (Desulfovibrio sp.), pseudomonas (Pseudomonas sp.), flavobacterium (Flavobacterium sp.), and micrococcus (Micrococcus sp.), which are paper/pulp-degrading microorganisms (including slime-forming bacteria); aspergillus (Aspergillus sp.), penicillium (Penicillium sp.), chaetomium (Chaetomium sp.), myrothecium (Myrothecium sp.), curvularia (Curvularia sp.), gliomastix (Gliomastix sp.), memnoniella (Memnoniella sp.), sarcopodium (Sarcopodium sp.), stachybotrys (Stschybotrys sp.), stemphylium (Stemphylium sp.), zygorhynchus (Zygorhynchus sp.), bacillus (Bacillus sp.), and staphylococcus (Staphylococcus sp.), which are fiber-degrading microorganisms; Fomitopsis palustris (Tyromyces palustris), C. versicolor (Coriolus versicolor), aspergillus (Aspergillus sp.), penicillium (Penicillium sp.), rhizopus (Rhizopus sp.), aureobasidium (Aureobasidium sp.), gliocladium (Gliocladum sp.), cladosporium (Cladosporium sp.), chaetomium (Chaetomium sp.), and trichoderma (Trichoderma sp.), which are wood-rotting microorganisms; aspergillus (Aspergillus sp.), penicillium (Penicillium sp.), chaetomium (Chaetomium sp.), cladosporium (Cladosporium sp.), mucor (Mucor sp.), paecilomyces (Paecilomyces sp.), pilobolus (Pilobus sp.), pullularia (Pullularia sp.), trichosporon (Trichosporon sp.), and trichothecium (Tricothecium sp.), which are leather-deteriorating microorganisms; aspergillus (Aspergillus sp.), penicillium (Penicillium sp.), rhizopus (Rhizopus sp.), trichoderma (Trichoderma sp.), chaetomium (Chaetomium sp.), myrothecium (Myrothecium sp.), streptomyces (Streptomyces sp.), pseudomonas (Pseudomonas sp.), bacillus (Bacillus sp.), micrococcus (Micrococcus sp.), serratia (Serratia sp.), margarinomyces (Margarinomyces sp.), and monascus (Monascus sp.), which are rubber-plastic degrading microorganisms; aspergillus (Aspergillus sp.), penicillium (Penicillium sp.), cladosporium (Cladosporium sp.), aureobasidium (Aureobasidium sp.), gliocladium (Gliocladium sp.), botryodiplodia (Botryodiplodia sp.), macrosporium (Macrosporium sp.), monilinia (Monilinia sp.), phoma (Phoma sp.), pullularia (Pullularia sp.), sporotrichum (Sporotrichum sp.), trichoderma (Trichoderma sp.), bacillus (bacillus sp.), proteus (Proteus sp.), pseudomonas (Pseudomonas sp.), and serratia (Serratia sp.), which are paint-deteriorating microorganisms.

### (2) Formulation

An industrial material protectant containing a pyrazole derivative (I) as an active ingredient may also contain various components in addition to the pyrazole derivative (I). An industrial material protectant containing the pyrazole derivative (I) as an active ingredient can be dissolved or dispersed in an appropriate liquid carrier or mixed with a solid carrier. The industrial material protectant containing the pyrazole derivative (I) as an active ingredient may further contain an emulsifier, a dispersant, a spreading agent, a penetration agent, a wetting agent, or a stabilizer, as needed. Furthermore, examples of the formulation type of the industrial material protectant containing the pyrazole derivative (I) as an active ingredient include wettable powders, powders, granules, tablets, pastes, suspending agents, and spray formulation . An industrial material protectant containing the pyrazole derivative (I) as an active ingredient may include other fungicides, insecticides, antidegradants, or the like.

The liquid carrier is not particularly limited provided that it does not react with the active ingredient. Examples of the liquid carrier include water, alcohols (for example, methyl alcohol, ethyl alcohol, ethylene glycol, and cellosolve), ketones (for example, acetone and methyl ethyl ketone), ethers (for example, dimethyl ether, diethyl ether, dioxane, and tetrahydrofuran), aromatic hydrocarbons (for example, benzene, toluene, xylene, and methyl naphthalene), aliphatic hydrocarbons (for example, gasoline, kerosene, lamp oil, machine oil, and fuel oil), amides (for example, dimethylformamide and N-methylpyrrolidone), halogenated hydrocarbons (for example, chloroform and carbon tetrachloride), esters (for example, ethyl acetate and glycerol esters of fatty acids), nitriles (for example, acetonitrile), and dimethyl sulfoxide.

Furthermore, fine powders or granules such as kaolin clay, bentonite, acid clay, pyrophyllite, talc, diatomaceous earth, calcite, urea, and ammonium sulfate can be used as the solid carrier.

Soaps, alkyl sulfonic acids, alkyl aryl sulfonic acids, dialkyl sulfosuccinates, quaternary ammonium salts, oxyalkylamines, fatty acid esters, and polyalkylene oxide-based and anhydrosorbitol-based surfactants can be used as the emulsifier and dispersant.

When the pyrazole derivative (I) is contained as an active ingredient in the formulation, the content proportion thereof depends on the type of agent and the purpose of use, and the content proportion may be 0.1 to 99.9 % wt.% with respect to the total mass of the formulation. In actual use, the treatment concentration thereof is preferably adjusted by adding a solvent, diluent, extender, etc., as appropriate, so that the concentration is usually from 0.005 to 5 wt.%, and preferably from 0.01 to 1 wt.%.

The formulation containing the pyrazole derivative (I) as an active ingredient exhibits, in particular, antifungal activity against plant pathogens. Thus, the formulation containing the pyrazole derivative (I) as an active ingredient can be suitably used as an antimicrobial agent for agricultural or horticultural use or for industrial use.
According to the composition described above, a high control efficacy can be exhibited against a wide range of plant diseases and plant pathogens. Such an effect contributes to achievement of Goal 2 "Zero hunger" of Sustainable Development Goals (SDGs) advocated by the United Nations, for example.

### [Summary]

A pyrazole derivative according to Aspect 1 of the present invention is a pyrazole derivative represented by General Formula (I) below, or an agrochemically acceptable salt thereof or an N-oxide thereof. 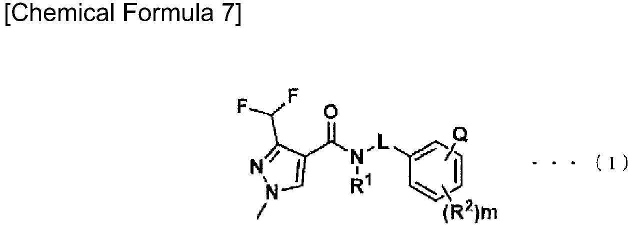 In General Formula (I),
Q is X-A-B;
X is a C₂-C₈ alkynylene group, a C₂-C₈ alkenylene group, or a C₁-C₈ alkylene group;
A is (i) -O-, -SO₂-, -COO-, or -NR^{Q}-, or (ii) a single bond or a linear C₁-C₄ alkylene group,
R^{Q} is hydrogen or a C₁-C₄ alkyl group,
B is a phenyl group, a benzyl group, a pyridyl group, a naphthyl group, a quinolinyl group, a pyridylmethyl group, or a tetrahydropyranyl group;
B is unsubstituted or substituted with n R^{B} substituents,
when A is (i), R^{B} is a halogen group, a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group;
when A is (ii), R^{B} is a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group;
n is 1, 2, 3, or 4, and when n is 2 or more, the plurality of R^{B} substituents are each independently selected;
R² is a halogen group, a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group; m is 0, 1, 2, 3, or 4, and when m is 2 or more, the plurality of R² substituents are each independently selected;
R¹ is hydrogen, a C₁-C₄ alkyl group, a C₃-C₆ cycloalkyl group, a C₁-C₄ alkoxy group, a C₁-C₄ haloalkyl group, or a hydroxy group; and
L is a C₁-C₆ alkylene group.

The pyrazole derivative according to Aspect 2 of the present invention is the pyrazole derivative described in Aspect 1, or an agrochemically acceptable salt thereof or an N-oxide thereof, where the pyrazole derivative is represented by General Formula (II) below.

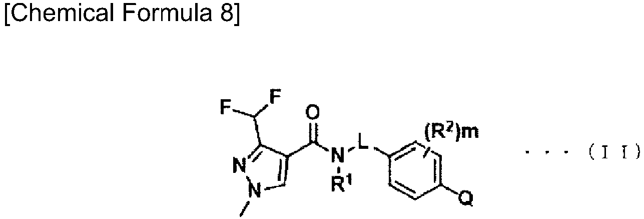

The pyrazole derivative according to Aspect 3 of the present invention is the pyrazole derivative, or the agrochemically acceptable salt thereof or the N-oxide thereof described in Aspect 1 or 2, where,
in General Formula (I) above,
X is a C₂-C₈ alkynylene group;
A is (i) -O-, -SO₂-, or -NR^{Q}-, or (ii) a single bond;
when A is (i), R^{B} is a halogen group, a C₁-C₄ alkyl group, a C₁-C₄ alkoxy group, a C₁-C₄ haloalkyl group, a C₁-C₄ haloalkoxy group, or an amino group;
when A is (ii), R^{B} is a C₁-C₄ alkyl group, a C₁-C₄ alkoxy group, a C₁-C₄ haloalkyl group, a C₁-C₄ haloalkoxy group, or an amino group; and
R² is a halogen group.

The pyrazole derivative according to Aspect 4 of the present invention is the pyrazole derivative, or the agrochemically acceptable salt thereof or the N-oxide thereof described in any one of Aspects 1 to 3, where,
in General Formula (I) above,
A is -O-;
B is a phenyl group, a benzyl group, or a pyridyl group;
R^{B} is a halogen group or a C₁-C₄ alkoxy group; and
R¹ is a C₁-C₄ alkoxy group.

The pyrazole derivative according to Aspect 5 of the present invention is the pyrazole derivative, or the agrochemically acceptable salt thereof or the N-oxide thereof described in any one of Aspects 1 to 4, where,
in General Formula (I) above,
when A is (i), n is 1 or 2;
when A is (ii), n is 1; and
m is 1 or 2.

The pyrazole derivative according to Aspect 6 of the present invention is the pyrazole derivative, or the agrochemically acceptable salt thereof or the N-oxide thereof described in any one of Aspects 1 to 5, where,
in General Formula (I) above,
L is a branched C₁-C₆ alkylene group.

An agricultural or horticultural chemical or industrial material protectant according to Aspect 7 of the present invention is an agricultural or horticultural chemical or industrial material protectant containing:
the pyrazole derivative, or the agrochemically acceptable salt thereof or the N-oxide thereof described in any one of Aspects 1 to 6 as an active ingredient.

Embodiments of the present invention will be further described in detail using the examples below. Needless to say, the present invention is not limited to the following examples, and details of the present invention can be in various manners. Furthermore, the present invention is not limited to the embodiments described above, and it may be varied in various ways within the scope of the claims. Thus, an embodiment achieved by appropriately combining technical means described herein will be included in the technical scope of the present invention.

### EXAMPLES

Examples of the present invention will be described below.

### [Synthesis of compounds]

### Synthesis Example 1

The compound a-1 was obtained according to the following synthesis scheme.

### Synthesis of Compound a-1

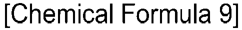

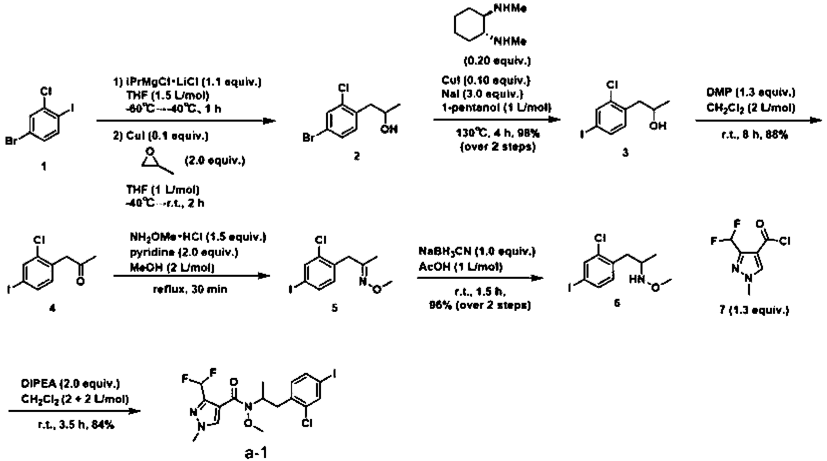

### Synthesis of Compound 2

In an argon atmosphere, 15.1 g of 4-bromo-2-chloro-1-iodobenzene and 71 mL of THF were added in a pear-shaped flask and cooled to -60°C, then 25 mL of 2.0 M iPrMgCl·LiCl in THF was added, and the mixture was stirred for 1 hour while the temperature was gradually raised to -40°C. After 907 mg of copper iodide was added to the reaction solution, 6.66 mL of propylene oxide and 48 mL of THF were added and stirred for 2 hours while the temperature was raised to room temperature. A saturated aqueous ammonium chloride solution was added thereto to terminate the reaction. After the mixture was extracted with ethyl acetate, the organic phase was washed with saturated brine. The obtained crude product was subjected to silica gel chromatography, and 12.3 g of a crude compound 2 was obtained as an orange oily substance.

### Synthesis of Compound 3

In an argon atmosphere, 12.3 g of the compound 2, 1.48 mL of trans-N,N'-dimethylcyclohexane-1,2-diamine, 907 mg of copper iodide, 21.4 g of sodium iodide, and 47.6 mL of 1-pentanol were added in a pear-shaped flask and stirred at 130°C for 4 hours. After the reaction solution was cooled, a saturated aqueous ammonium chloride solution was added. The mixture was extracted with ethyl acetate, followed by washing with saturated brine. The obtained crude product was subjected to silica gel chromatography, and 13.8 g of a compound 3 was obtained as an orange oily substance. The yield was 98% (over 2 steps).

### Synthesis of Compound 4

In an argon atmosphere, 13.8 g of the compound 3 and 93 mL of dichloromethane were added in a pear-shaped flask, and then 25.7 g of Dess-Martin periodinane was added. The mixture was stirred at room temperature for 8 hours. A saturated aqueous sodium hydrogencarbonate solution and 20% aqueous sodium thiosulfate solution were added thereto to terminate the reaction, and then the mixture was extracted with ethyl acetate, followed by washing with saturated brine. The obtained crude product was subjected to silica gel chromatography, and 12.0 g of a compound 4 was obtained as a yellow oily substance. The yield was 88%.

### Synthesis of Compound 5

In a pear-shaped flask, 12.0 g of the compound 4, 5.10 g of O-methylhydroxylamine hydrochloride, 6.57 mL of pyridine, and 81.4 mL of MeOH were added and heated at 70°C under reflux for 30 minutes. After the solvent was distilled off under reduced pressure by using an evaporator, ethyl acetate was added, followed by washing with water and saturated brine, and 12.9 g of a crude compound 5 was obtained as a yellow oily substance.

### Synthesis of Compound 6

In an argon atmosphere, 12.9 g of the compound 5 and 39.9 mL of acetic acid were added in a pear-shaped flask, and then 2.51 g of sodium cyanoborohydride was added. The mixture was stirred at room temperature for 1.5 hours. After 6 M aqueous sodium hydroxide solution was added, the mixture was extracted with ethyl acetate, the organic phase was washed with water and saturated brine, and thus 12.7 g of a compound 6 was obtained as a colorless oily substance. The yield was 96% (over 2 steps).

### Synthesis of Compound a-1

In an argon atmosphere, 8.15 g of 3-difluoromethyl-1-methylpyrazole-4-carboxylic acid, 71 mL of dichloromethane, and 6.32 g of oxalyl chloride were added in a pear-shaped flask, and then a few drops of N,N-dimethylformamide were added. The mixture was stirred at room temperature for 3 hours. The solvent was distilled off from the reaction solution, and thus a crude compound 7 was prepared.

In another pear-shaped flask, 11.6 g of the compound 6, 36 mL of dichloromethane, and 9.20 g of DIPEA were added, then the compound 7 dissolved in 36 mL of dichloromethane was slowly added, and the mixture was stirred at room temperature for 3.5 hours. A saturated aqueous sodium hydrogencarbonate solution was added thereto to terminate the reaction, the mixture was extracted with ethyl acetate, followed by washing with saturated brine. The obtained fraction was dissolved in 50 mL of chloroform, then 100 mL of hexane was added, and the mixture was allowed to stand in a refrigerator overnight. The precipitated solid was filtered to remove the filtrate, and 14.4 g of a compound a-1 was obtained as a white solid. The yield was 84%.

### Compound I-16

Compound I-16 was obtained according to the following scheme. Note that the following scheme corresponds to step 1 of the synthesis scheme 1 described above.

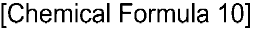

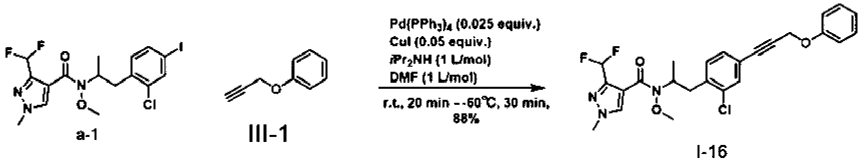

### Synthesis of Compound I-16

In an argon atmosphere, 5.51 g of the compound a-1, 109 mg of copper iodide, 329 mg of tetrakis(triphenylphosphine)palladium(0), 11.4 mL of diisopropylamine, and 11.4 mL of N,N-dimethylformamide were added in a pear-shaped flask. 2.26 g of the compound III-1 was added thereto, and the mixture was allowed to react at room temperature for 20 minutes. The temperature was raised to 60°C, and the mixture was further stirred for 30 minutes. A saturated aqueous ammonium chloride solution was added thereto to terminate the reaction. The mixture was extracted with ethyl acetate, followed by washing with water and saturated brine. The organic phase was distilled to remove the solvent and a crude product was obtained. The crude product was subjected to silica gel chromatography. The obtained fraction was dissolved in 25 mL of chloroform, then 50 mL of hexane was added, and the mixture was allowed to stand in a refrigerator overnight. The precipitated solid was filtered to remove the filtrate, and thus 4.89 g of a compound I-16 was obtained as a white solid. The yield was 88%.
¹H NMR (CDCl)₃ 7.40 (m, 2H), 7.31 (m, 3H), 7.21 (m, 2H), 7.00 (m, 3H), 4.88 (s, 2H), 4.68 (m, 1H), 3.92 (s, 3H), 3.58 (s, 3H), 3.21 (m, 1H), 3.04 (m, 1H), 1.41 (d, J = 6.9 Hz, 3H).

### Compound I-54

A compound I-54 was obtained according to the following scheme. Note that the following scheme corresponds to step 2 of the synthesis scheme 1 described above.

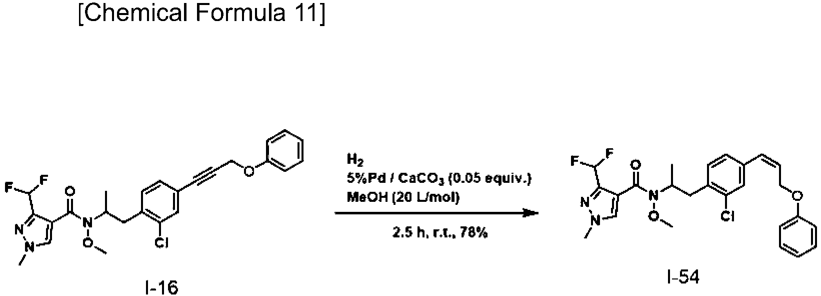

### Synthesis of Compound I-54

In a pear-shaped flask, 171 mg of the compound I-16, 10 mL of methanol, and 53.2 mg of 5% Pd/CaCO₃ were added, and the mixture was stirred in a hydrogen atmosphere at room temperature for 2.5 hours. After the catalyst was removed by Celite filtration, the mixture was distilled to remove the solvent and a crude product was obtained. The crude product was subjected to silica gel chromatography, and thus 191 mg of a compound I-54 was obtained as a white solid. The yield was 78%.
¹H NMR (CDCl)₃ 7.63 (s, 1H), 7.28 (m, 3H), 7.23 (m, 1H), 7.18 (m, 1H), 7.04 (d, J = 6.9 Hz, 1H), 6.95 (t, J = 7.6 Hz, 1H), 6.87 (m, 2H), 6.57 (d, J = 11.7 Hz, 1H), 5.99 (m, 1H), 4.73 (dd, J = 6.2, 1.4 Hz, 1H), 4.68 (m, 1H), 3.91 (s, 3H), 3.60 (s, 3H), 3.23 (dd, J = 13.1, 8.3 Hz, 1H), 3.06 (dd, J = 13.1, 6.2 Hz, 1H), 1.42 (d, J = 6.9 Hz, 3H).

### Synthesis Example 2 (Synthesis Scheme 1)

### Compound I-44

A compound I-44 was obtained according to the following scheme. Note that the following scheme corresponds to step 1 of the synthesis scheme 1 described above.

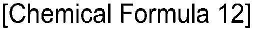

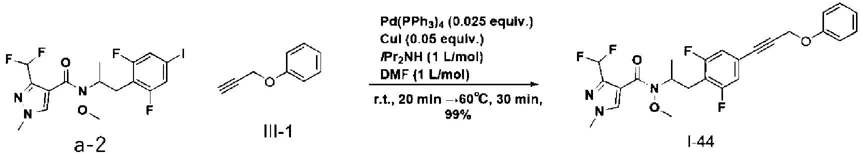

### Synthesis of Compound I-44

In an argon atmosphere, 485 mg of the compound a-2, 9.52 mg of copper iodide, 28.9 mg (0.025 mmol, 0.025 equiv.) of tetrakis(triphenylphosphine)palladium(0), 1.0 mL of diisopropylamine, and 1.0 mL of N,N-dimethylformamide were added in a pear-shaped flask. 198 mg of the compound III-1 was added thereto, and then the mixture was allowed to react at room temperature for 20 minutes. The temperature was raised to 60°C, and the mixture was further stirred for 30 minutes. A saturated aqueous ammonium chloride solution was added thereto to terminate the reaction. The mixture was extracted with ethyl acetate, followed by washing with water and saturated brine. The organic phase was distilled to remove the solvent and a crude product was obtained. The crude product was subjected to silica gel chromatography, and thus 484 mg of a compound I-44 was obtained as a brown solid. The yield was 99%.
¹H NMR (CDCl)₃ 7.74 (s, 1H), 7.31 (m, 2H), 7.31 (m, 3H), 7.13 (m, 1H), 7.00 (m, 3H), 6.88 (d, J = 6.9 Hz, 2H), 4.87 (s, 2H), 4.79 (m, 1H), 3.95 (s, 3H), 3.67 (s, 3H), 3.13 (m, 1H), 2.92 (m, 1H), 1.38 (d, J = 6.9 Hz, 3H).

### Compound I-56

A compound I-56 was obtained according to the following scheme. Note that the following scheme corresponds to step 3 of the synthesis scheme 1 described above.

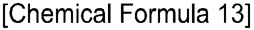

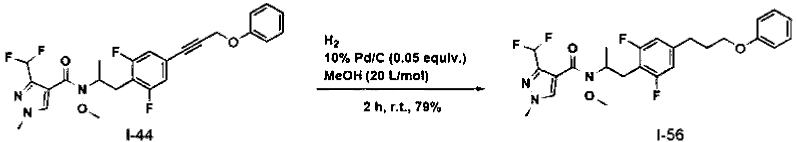

### Synthesis of Compound I-56

In a pear-shaped flask, 484 mg of the compound I-44 and 19.8 mL of methanol were added, and then 95.8 mg of 10% Pd/C was added, and the mixture was stirred in a hydrogen atmosphere at room temperature for 2 hours. After the catalyst was removed by Celite filtration, the mixture was distilled to remove the solvent and a crude product was obtained. The crude product was subjected to silica gel chromatography, and thus 385 mg of a compound I-56 was obtained as a white solid. The yield was 79%.
¹H NMR (CDCl)₃ 7.77 (s, 1H), 7.27 (m, 2H), 7.13 (t, J = 54.4 Hz, 1H), 6.94 (t, J = 7.6 Hz, 1H), 6.87 (d, J = 7.6 Hz, 2H), 6.67 (m, 2H), 4.80 (m, 1H), 3.95 (s, 3H), 3.91 (t, J = 6.2 Hz, 2H), 3.68 (s, 3H), 3.09 (dd, J = 13.1, 7.6 Hz, 1H), 2.90 (dd, J = 13.1, 6.9 Hz, 1H), 2.73 (t, J = 8.3 Hz, 2H), 2.02 (m, 2H), 1.38 (d, J = 6.9 Hz, 3H).

### Synthesis Example 3 (Synthesis Scheme 2)

A compound I-52 was obtained according to the following scheme. Note that the following scheme corresponds to the synthesis scheme 2 described above.

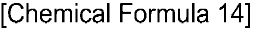

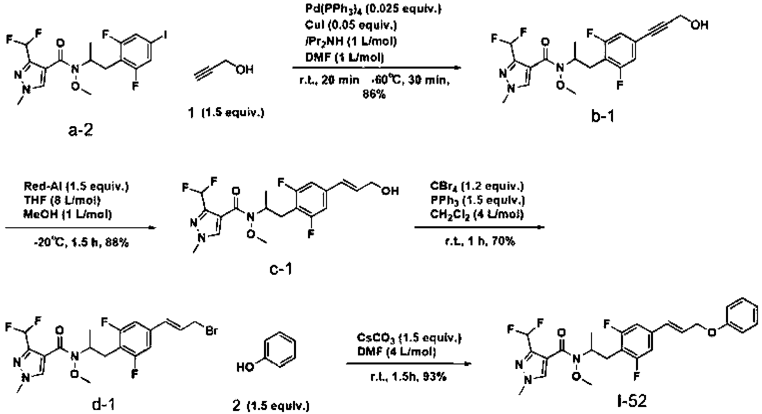

### Synthesis of Compound b-1

In an argon atmosphere, 1.46 g of the compound a-2, 28.6 mg of copper iodide, 86.7 mg of tetrakis(triphenylphosphine)palladium(0), 3.0 mL of diisopropylamine, and 3.0 mL of N,N-dimethylformamide were added in a pear-shaped flask. 252 mg of the compound 1 was added thereto, and the mixture was allowed to react at room temperature for 20 minutes. The temperature was raised to 60°C, and the mixture was further stirred for 30 minutes. A saturated aqueous ammonium chloride solution was added thereto to terminate the reaction. The mixture was extracted with ethyl acetate, followed by washing with water and saturated brine. The organic phase was distilled to remove the solvent and a crude product was obtained. The crude product was subjected to silica gel chromatography, and thus 1.07 g of a compound b-1 was obtained as a yellow oily substance. The yield was 86%.

### Synthesis of Compound c-1

In an argon atmosphere, 740 mg of the compound b-1 and 14.3 mL of THF were added in a pear-shaped flask and then cooled to -20°C. 0.75 mL of Red-Al (70 wt% toluene solution) was added, and the mixture was stirred for 1.5 hours while the temperature was being returned to room temperature. 17.9 mL of 1 N aqueous potassium sodium tartrate solution was added thereto to terminate the reaction, and the mixture was extracted with ethyl acetate. The organic phase was distilled to remove the solvent and a crude product was obtained. The crude product was subjected to silica gel chromatography, and thus 654 mg of a compound c-1 was obtained as a white solid. The yield was 88%.

### Synthesis of Compound d-1

In an argon atmosphere, 496 mg of the compound c-1 and 4.8 mL of dichloromethane were added in a pear-shaped flask and then cooled to 0°C. 474 mg of carbon tetrabromide and 469 mg of triphenylphosphine were added and the mixture was stirred at room temperature for 1 hour. The reaction solution was distilled to remove the solvent and a crude product was obtained. The crude product was subjected to silica gel chromatography, and thus 400 mg of a compound d-1 was obtained as a white solid. The yield was 70%.

### Synthesis of Compound I-52

In an argon atmosphere, 148 mg of the compound d-1 and 1.24 mL of DMF were added in a pear-shaped flask, and then 43.8 mg of the compound 2 and 152 mg of cesium carbonate were added. The mixture was stirred at room temperature for 1.5 hours. Water was added, and the mixture was extracted with ethyl acetate, followed by washing with saturated brine. The organic phase was distilled to remove the solvent and a crude product was obtained. The crude product was subjected to silica gel chromatography, and thus 142 mg of a compound I-52 was obtained as a white solid. The yield was 93%. ¹H NMR (CDCl)₃ 7.78 (s, 1H), 7.28 (m, 2H), 7.13 (t, J = 54.4 Hz, 1H), 6.96 (t, J = 6.9 Hz, 1H), 6.87 (d, J = 9.0 Hz, 2H), 6.72 (d, J = 8.3 Hz, 2H), 6.53 (d, J = 11.7 Hz, 1H), 6.01 (m, 1H), 4.83 (m, 1H), 4.71 (d, J = 6.2 Hz, 2H), 3.94 (s, 3H), 3.69 (s, 3H), 3.14 (dd, J = 13.1, 7.6 Hz, 1H), 2.96 (dd, J = 13.1, 6.9 Hz, 1H), 1.40 (d, J = 6.9 Hz, 3H).

### Synthesis Example 4. Synthesis of Other Compounds

Compounds corresponding to compound Nos. I-1 to I-15, I-17 to I-43, I-45 to I-51, I-53, I-55, and I-57 to I-90 were synthesized by appropriately changing the compound used, conditions, and the like in Synthesis Examples 1 to 3 described above.

NMR measurement data for each of the synthesized compounds are shown in Table 2.

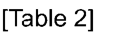

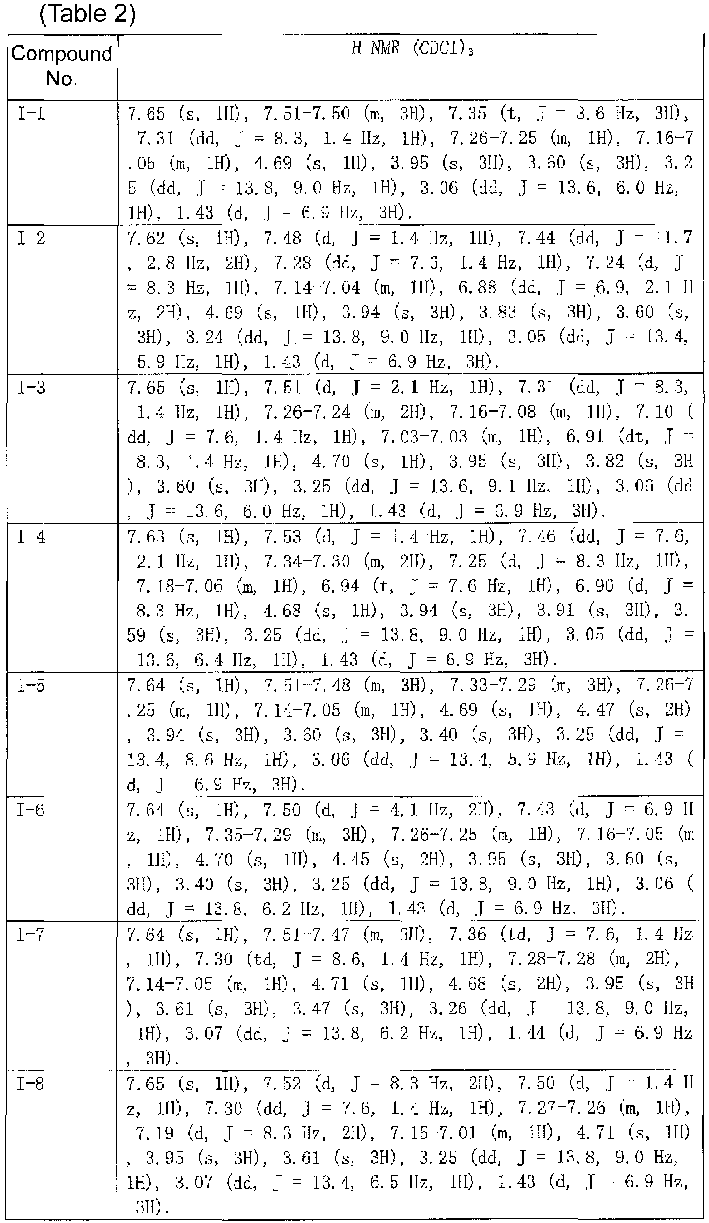

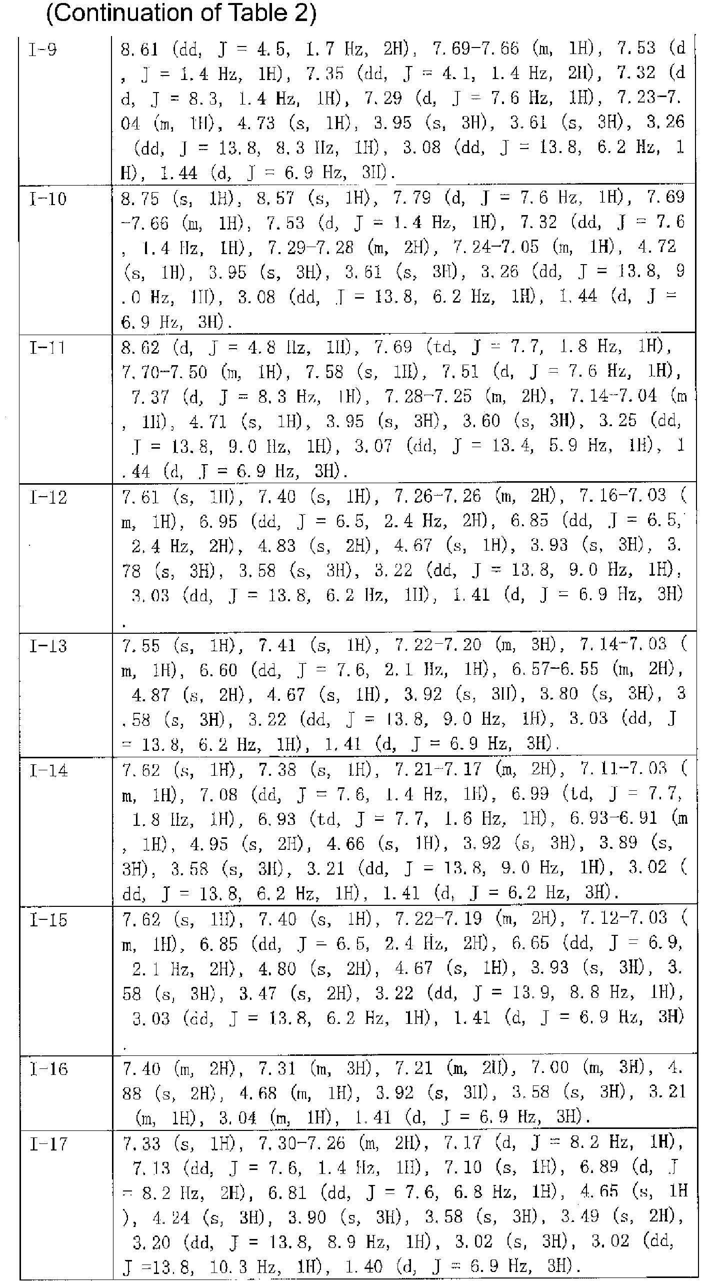

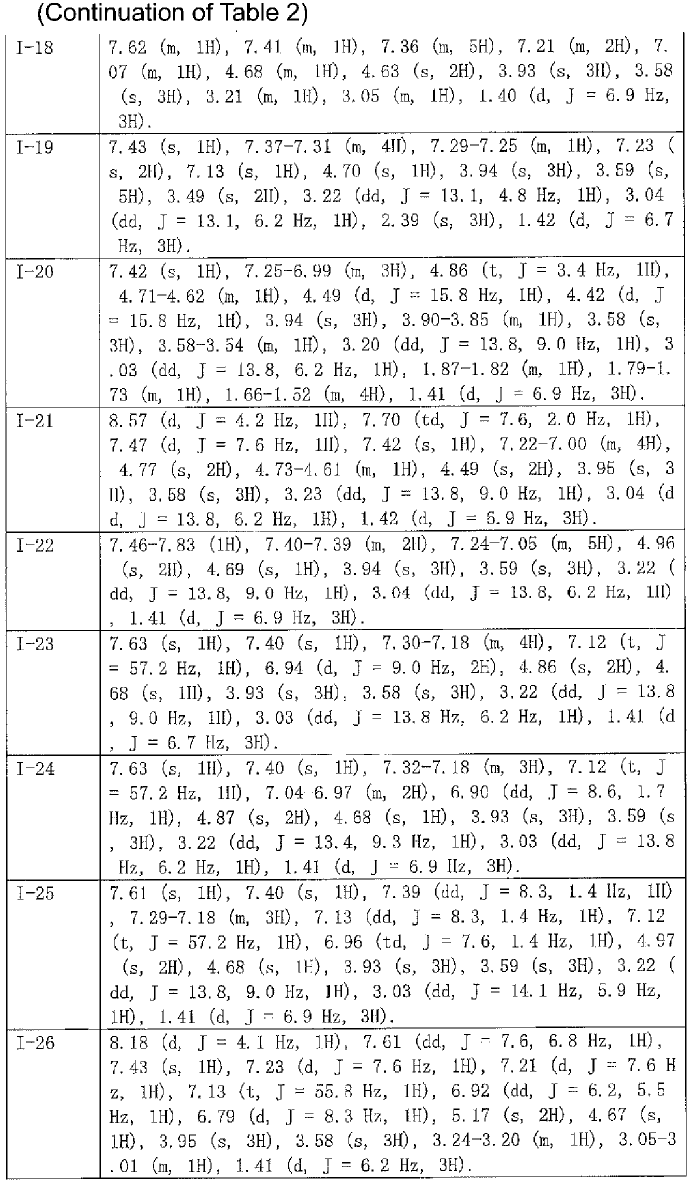

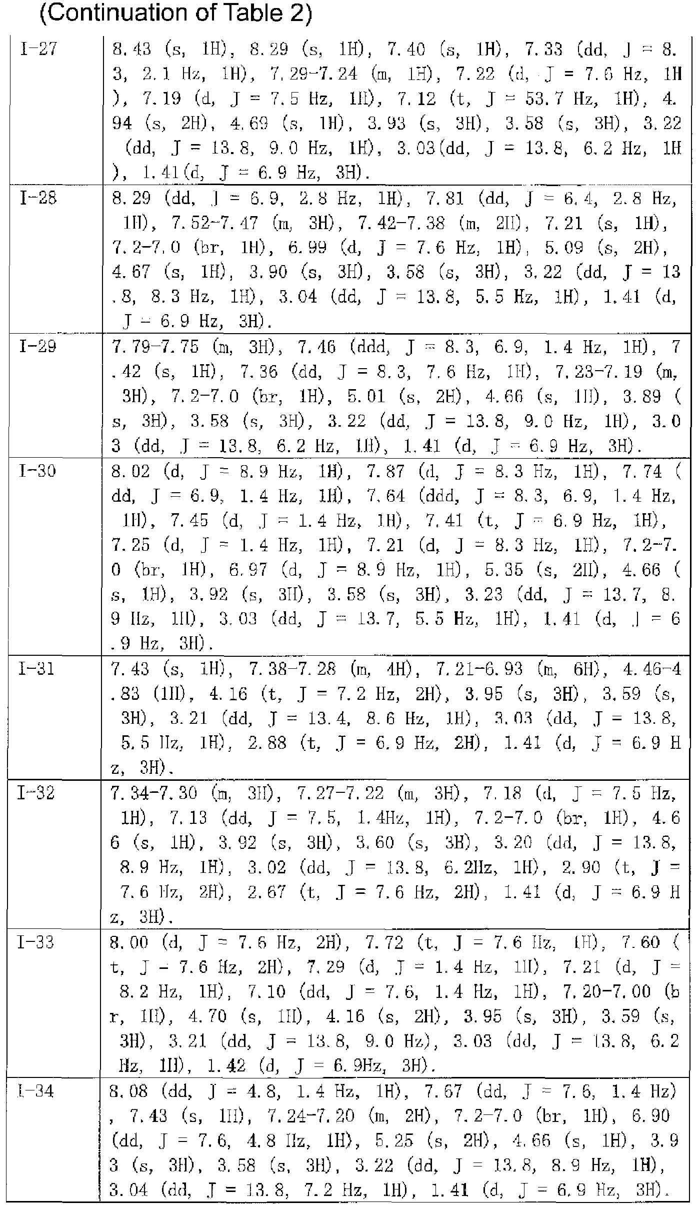

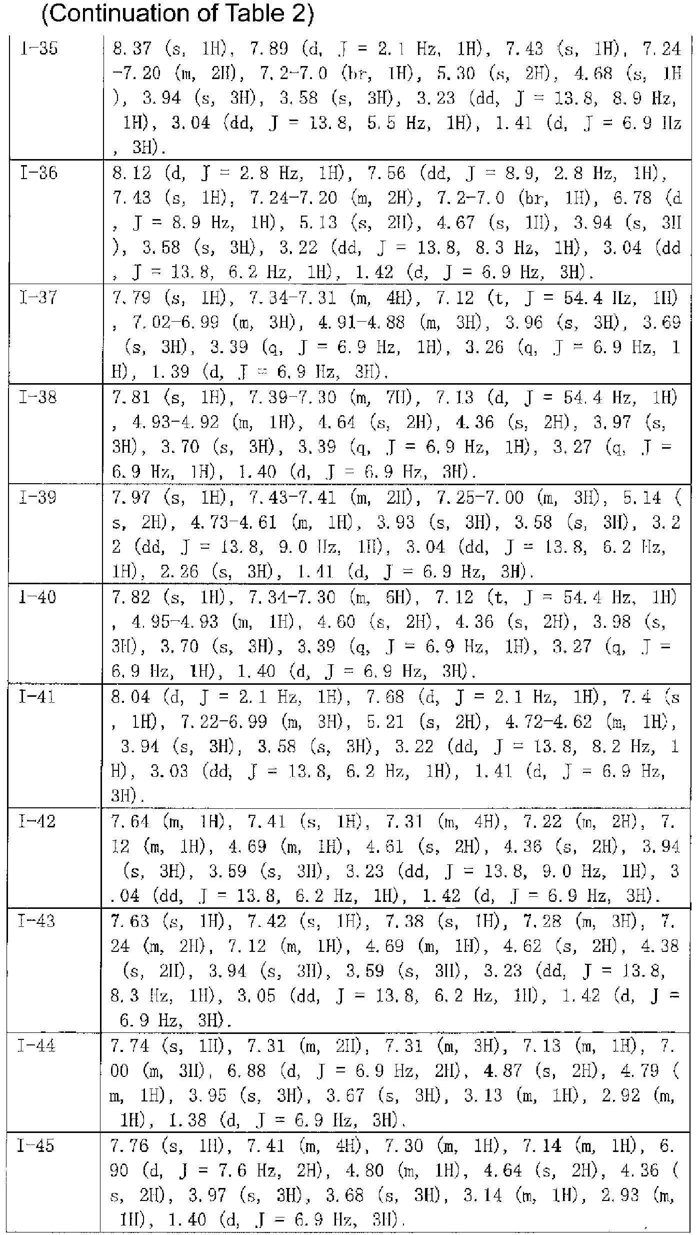

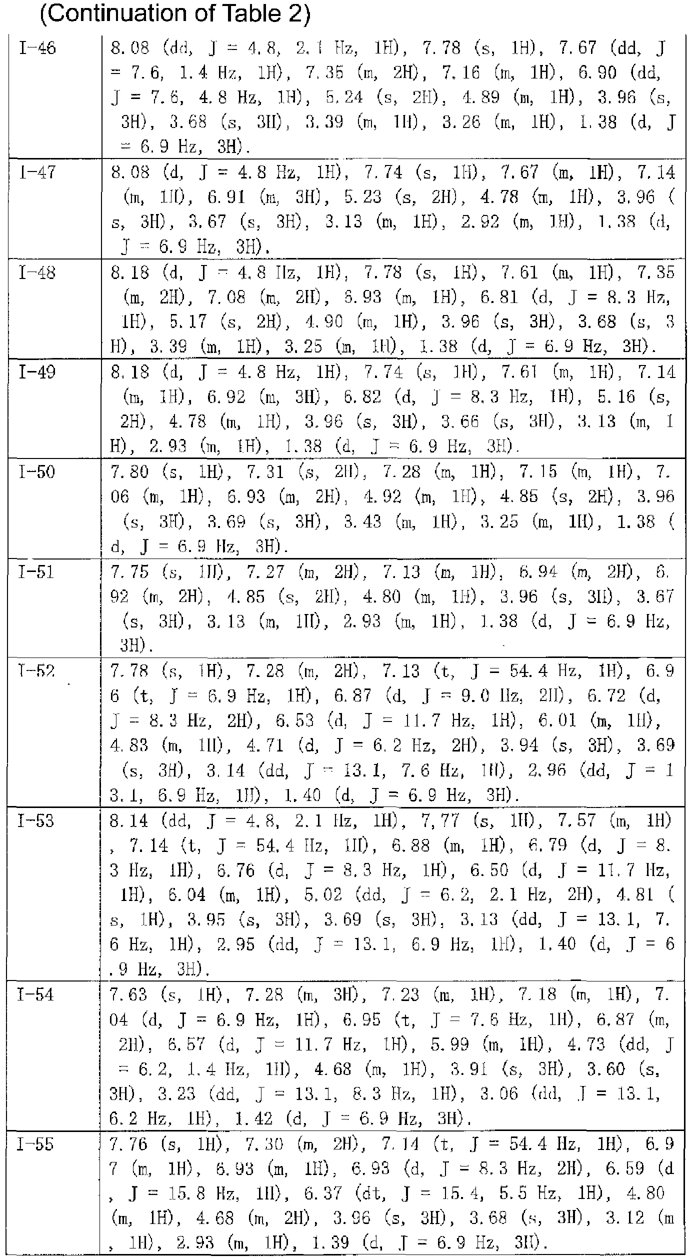

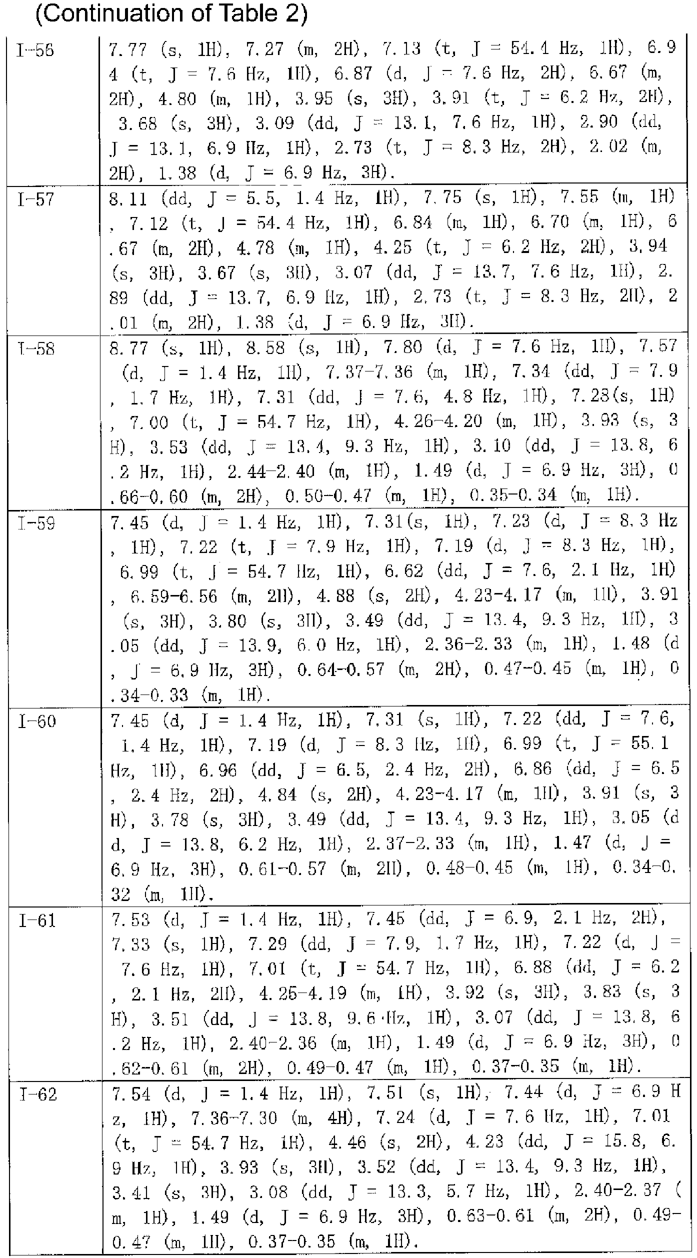

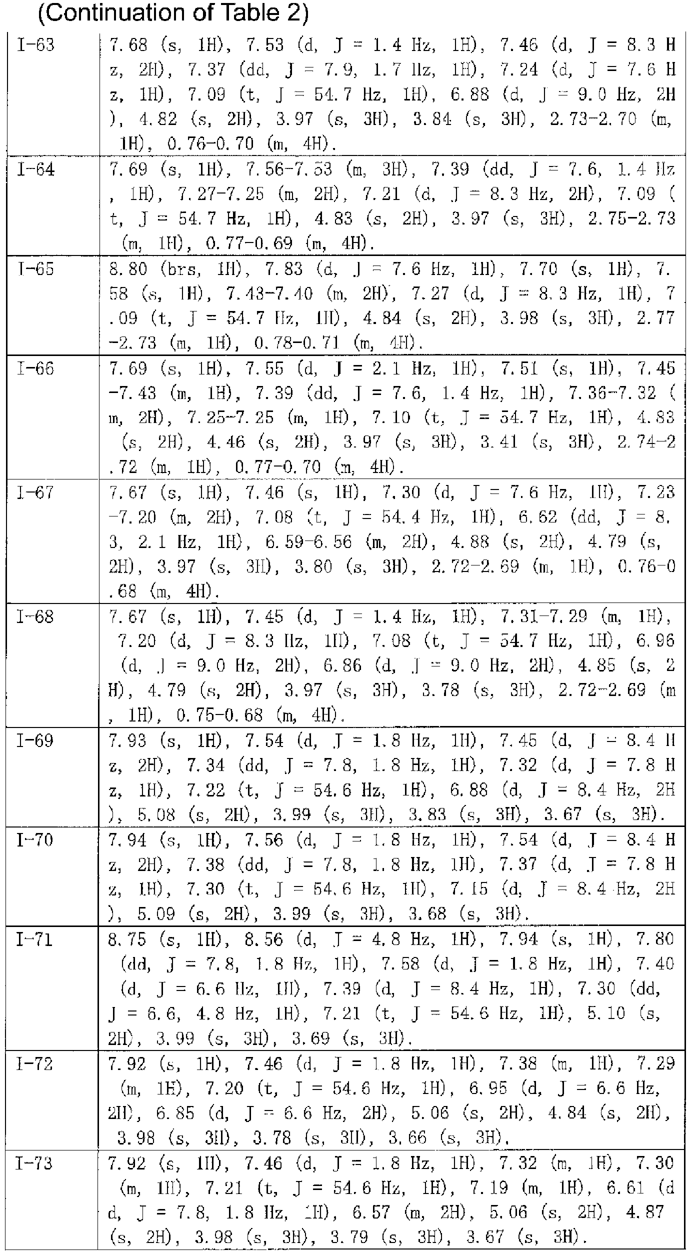

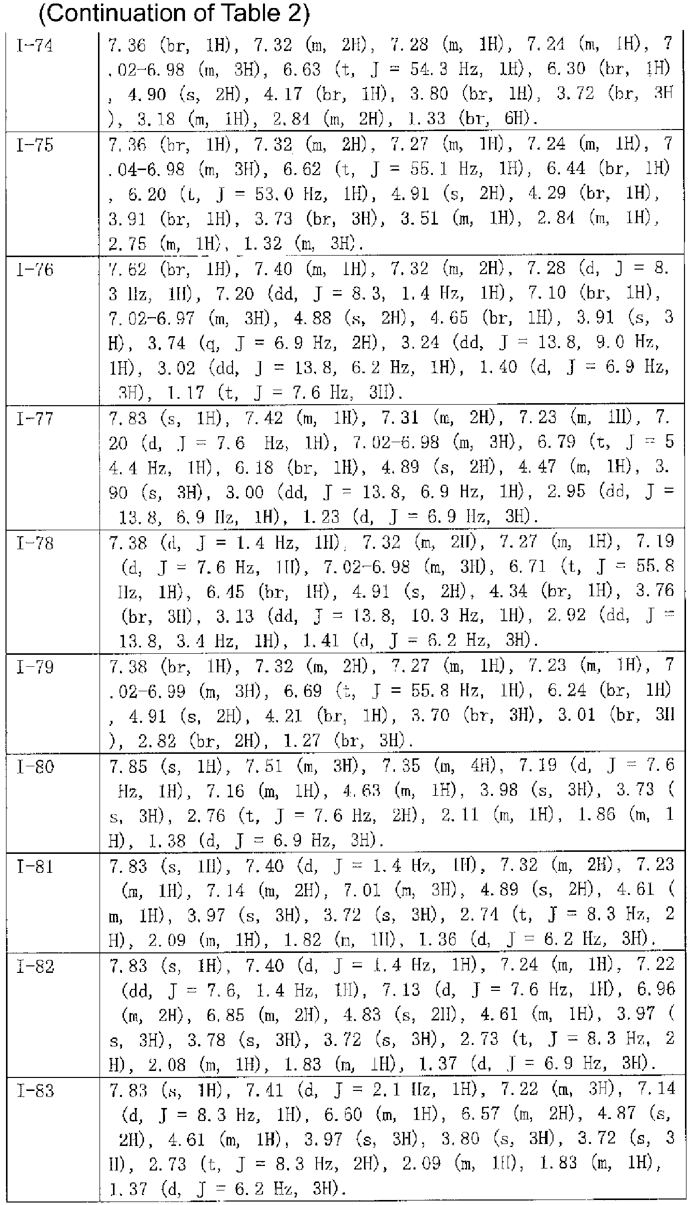

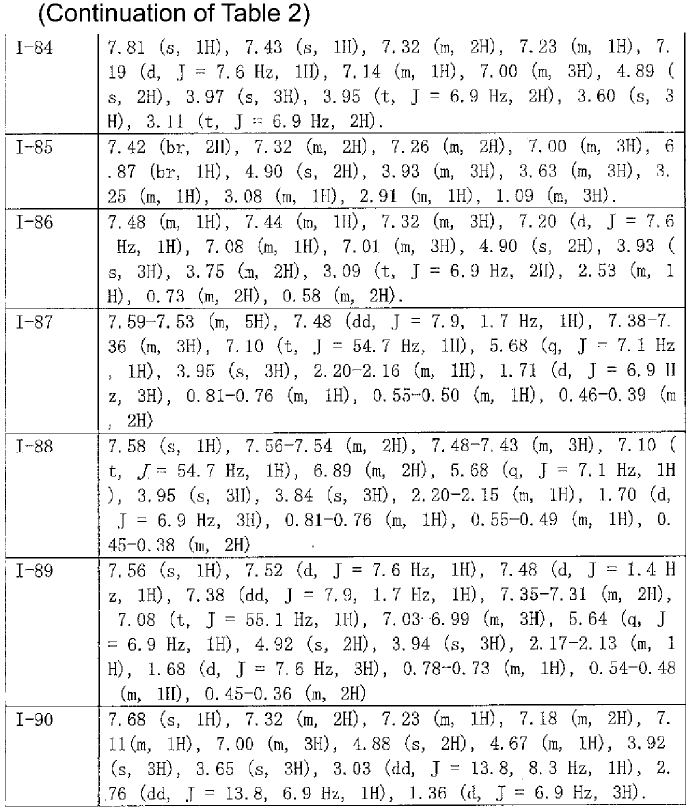

### Formulation Example

Emulsions were formulated as follows using any synthesized pyrazole derivative described in this specification.

### Formulation Example 1 (Emulsion)

| | |
|---|---|
| Pyrazole derivative | 2.6 parts |
| Polyoxyalkylene allylphenyl ether · alkylbenzenesulfonic acid metal salt · xylene mixture | 15.0 parts |
| Cyclohexanone | 33.0 parts |
| Solvent naphtha | 49.4 parts |

The above components were uniformly mixed and dissolved to form an emulsion.

### Test Example

### Test Example 1: Antifungal activity test against plant pathogenic fungi

The antifungal action of the compound according to an embodiment of the present invention against various plant pathogenic filamentous fungi was tested by a Petri dish test.

The compound according to an embodiment of the present invention was dissolved in dimethyl sulfoxide to a predetermined concentration and added to a PDA culture medium (potato dextrose agar medium) sterilized by autoclave and then cooled to around 60°C in an amount of 1% (V/V). The culture medium was poured into the Petri dish and a plate culture medium containing the compound of an embodiment of the present invention was prepared by mixing thoroughly so that the chemical concentration in the PDA culture medium was uniform.

On the other hand, the fungal colony of various plant pathogenic microorganisms previously cultured on the PDA culture medium was punched out using a cork borer having a diameter of 4 mm, and inoculated into the aforementioned chemical containing plate culture medium. After culture was performed at a predetermined temperature for a predetermined period of time according to Table 3, the colony diameter on the chemical-treated plate was measured. The mycelial growth inhibition rate (%) was calculated by the following formula in comparison with a diameter of the fungal colony on the untreated plate containing no chemical. $R = 100 \left(dc-dt\right) / dc$ (where R = mycelial growth inhibition rate (%), dc = colony diameter on untreated plate, dt = colony diameter on chemical-treated plate.)
A larger mycelial growth inhibition rate indicates better antimicrobial action.

### [Table 3]

**(Table 3)**

| Pathogenic fungus name | Species | Culture temperature (°C) | Culture days (days) |
|---|---|---|---|
| Wheat leaf blight fungi | *Zymoseptoria tritici* | 20 °C | Day 14 |
| Wheat Fusarium head blight fungi | *Fusarium graminearum* | 25 °C | Day 3 |
| Citrus blue mold fungi | *Pepicillium italicum* | 25 °C | Day 3 |

### Test Example 1-1: Wheat Leaf Blight (Zymoseptoria tritici)

An antifungal test was performed using a wheat leaf blight fungi according to the aforementioned method. The compounds I-1 to I-4, I-8 to I-10, I-13, I-14, I-16 to I-32, I-34, I-35, I-37, I-38, I-40 to I-48, I-50, I-52 to I-57, I-61, I-63, I-64, I-67, I-68, I-77, and I-86 to I-89 each exhibited a mycelial growth inhibition rate of 80% or higher at 100 mg/L.

### Test Example 1-2: Wheat Fusarium Head Blight (Fusarium graminearum)

An antifungal test was performed using a wheat Fusarium head blight fungi according to the aforementioned method. The compounds I-1 to I-11, I-13, I-14, I-16 to I-32, I-34, I-35, I-37, I-38, I-40 to I-57, I-84, and I-87 each exhibited a mycelial growth inhibition rate of 70% or higher at 100 mg/L. Among these, the compounds I-1 to I-10, I-13, I-14, I-16 to I-26, I-28, I-30 to I-32, I-34, I-35, I-37, I-38, I-40 to I-50, and I-52 to I-57 each exhibited a mycelial growth inhibition rate of 80% or higher.

### Test Example 1-3: Citrus Blue Mold (Penicillium italicum)

An antifungal test was performed using a citrus blue mold fungi according to the aforementioned method. The compounds I-18 and I-41 each had a mycelial growth inhibition rate of 80% or higher at 100 mg/L.

### Test Example 2: Cucumber Gray Mold (Botrytis cinerea) Control Efficacy Test

The compound according to an embodiment of the present invention was dissolved in acetone containing 60 ppm of Gramin S, 5% (V/V) of this solution was added to water containing 60 ppm of Gramin S in a manner that the concentration became 100 g ai/ha, and this liquid was sprayed on cucumbers in the cotyledon stage (variety: hanjiro fushinari) cultivated using a square plastic pot (6.5 cm × 6.5 cm) at a ratio of 1000 L/ha. After the sprayed leaves were air-dried, a paper disc (8 mm in diameter) impregnated with spore liquid of gray mold was placed and kept under high-humidity conditions of 20°C. On day 4 after inoculation, a disease spot area ratio of cucumber gray mold was investigated and a control efficacy was calculated according to the following formula. 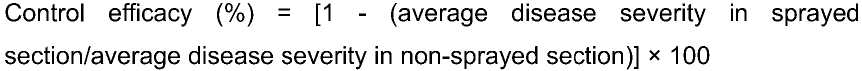 Control efficacy (%) = [1 - (average disease severity in sprayed section/average disease severity in non-sprayed section)] × 100

Further, the disease severity was determined based on Table 4 below.

### [Table 4]

**(Table 4)**

| Disease severity | Disease spot area ratio (%) |
|---|---|
| 0 | 0 |
| 0. 1 | < 5 |
| 0.5 | <10 |
| 1 | < 20 |
| 2 | < 40 |
| 3 | < 60 |
| 4 | < 80 |
| 5 | ≤ 100 |

In the tests described above, the compounds I-1 to I-8, I-10, I-12 to I-14, I-16 to I-18, I-20 to I-25, I-28, I-30, I-32, I-34 to I-51, I-55 to I-57, I-77, I-78, I-84, and I-86 each exhibited a control efficacy of 80% or higher.

### Test Example 3: Wheat Powdery Mildew (Blumeria graminis) Control Efficacy Test

Wheat (variety: Norin No. 61) was cultivated from the first to second leaf stage using a square plastic pot (6.5 cm × 6.5 cm). The compound according to an embodiment of the present invention was dissolved in acetone containing 60 ppm of Gramin S, 5% (V/V) of this solution was added to water containing 60 ppm of Gramin S in a manner that the concentration became 100 g ai/ha, and this liquid was sprayed on wheat at a ratio of 1000 L/ha. The sprayed liquid on the plant body was air-dried, and then the spore of wheat powdery mildew from wheat powdery mildew seedlings was sprinkled on and inoculated. Subsequently, it was managed in a greenhouse. On day 6 to day 8 after inoculation, the disease spot area ratio of wheat powdery mildew was investigated, and the control efficacy was calculated in the same manner as in Test Example 2.

In the tests described above, the compounds I-1, I-4, I-8, and I-18 each exhibited a control efficacy of 80% or higher.

### Test Example 4: Wheat Leaf Blight (Zymoseptoria tritici) Control Efficacy Test

Wheat (variety: Norin No. 61) was cultivated from the first to second leaf stage using a square plastic pot (6.5 cm × 6.5 cm). The compound according to an embodiment of the present invention was dissolved in acetone containing 60 ppm of Gramin S, 5% (V/V) of this solution was added to water containing 60 ppm of Gramin S in a manner that the concentration became 100 g ai/ha, and this liquid was sprayed on wheat at a ratio of 1000 L/ha. After the sprayed liquid on the plant body was air-dried, the spore of wheat leaf blight fungi (adjusted to 1 × 10⁷/mL, with Gramin S added to 60 ppm) was spray-inoculated, and kept at 20°C under high-humidity conditions for 72 hours. Subsequently, it was managed in an artificial weather chamber. On day 27 to day 33 after inoculation, the disease spot area ratio of wheat leaf blight was investigated, and the control efficacy was calculated in the same manner as in Test Example 2.

In the tests described above, the compounds I-1 to I-11, I-13, I-14, I-16, I-18, I-20, I-22 to I-26, I-28 to I-31, I-34, I-35, I-37, I-38, I-40 to I-50, I-52, I-53, I-55 to I-57, I-61, I-63 to I-67, I-75 to I-77, I-79, I-80, and I-86 each exhibited a control efficacy of 80% or higher.

### Test Example 5: Soybean Rust (Phakopsora pachyrhizi) Control Efficacy Test

Soybean (variety: Enrei) was cultivated from the first to second compound leaf development stage using a square plastic pot (6.5 cm × 6.5 cm). The compound according to an embodiment of the present invention was dissolved in acetone containing 60 ppm of Gramin S, 5% (V/V) of this solution was added to water containing 60 ppm of Gramin S in a manner that the concentration became 100 g ai/ha, and this liquid was sprayed on soybean at a ratio of 1000 L/ha. After the sprayed liquid on the plant body was air-dried, the spore of soybean rust fungi (adjusted to 1 × 10⁶/mL, with Gramin S added to 60 ppm) was spray-inoculated, and kept at 25°C under high-humidity conditions for 24 hours. Subsequently, it was managed in a greenhouse. On day 13 to day 15 after inoculation, the disease spot area ratio of soybean rust was investigated, and the control efficacy was calculated in the same manner as in Test Example 2.

In the tests described above, the compounds I-19, I-22 to I-25, and I-60 each exhibited a control efficacy of 80% or higher.

### Test Example 6: Comparison Test of Activity

A control compound A represented by General Formula A below was prepared.

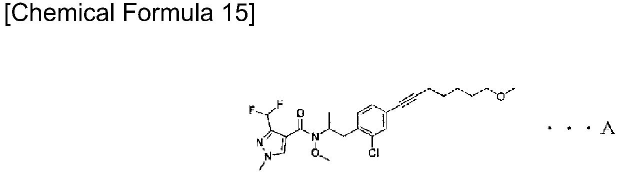

For the compounds I-16, I-18, and I-48 and the control compound A, the antifungal activity test against plant pathogenic fungi and the wheat leaf blight (Zymoseptoria tritici) control efficacy test were performed in the same manner as in Test Example 1-1 and Test Example 4 except for changing the concentration to a predetermined concentration. The results (mycelial growth inhibition rate (%) and control efficacy (%)) are presented in Table 5.

### [Table 5]

**(Table 5)**

| Test method | Concentration | 1-16 | 1-18 | I-48 | Control compound A |
|---|---|---|---|---|---|
| Antifungal activity test | 12.5 ppm | 97 | 95 | 100 | 71 |
| | 1.56 ppm | 80 | 79 | 72 | 58 |
| Control efficacy test | 12.5 ppm | 100 | 100 | 100 | 52 |

In a wide range of concentrations, the compounds I-16, I-18, and I-48 exhibited superior control efficacy against wheat leaf blight, compared to the control compound A.

### Test Example 7: Comparison Test of Activity

A control compound B represented by General Formula B below and a control compound C represented by General Formula C below were prepared.

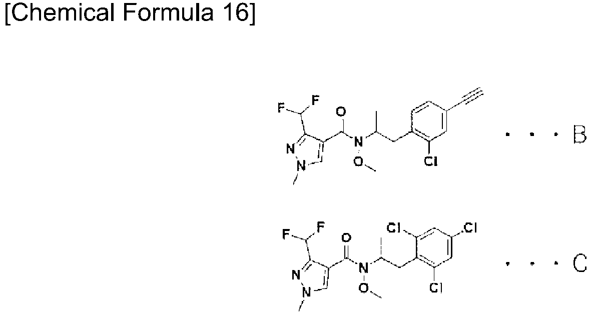

### Test Example 7-1

For the compound I-16, the control compound B, and the control compound C, the cucumber gray mold (Botrytis cinerea) control efficacy test was performed in the same manner as in Test Example 2 except for changing the concentration to a predetermined concentration. The results (control efficacy (%)) are presented in Table 6.

### [Table 6]

**(Table 6)**

| Test method | Concentration | I-16 | Control compound B | Control compound C |
|---|---|---|---|---|
| Control efficacy test | 100 ppm | 100 | 65 | 97 |
| | 12.5 ppm | 90 | 20 | 65 |

In a wide range of concentrations, the compound I-16 exhibited superior control efficacy against the cucumber gray mold, compared to the control compound B and the control compound C.

### Test Example 7-2

For the compound I-18 and the control compound C, the antifungal activity test against the citrus blue mold fungi (Botrytis cinerea) was performed in the same manner as in Test Example 1 except for changing the concentration to a predetermined concentration. The results (mycelial growth inhibition rate (%)) are presented in Table 7.

### [Table 7]

**(Table 7)**

| Test method | Concentration | I-I8 | Control compound C |
|---|---|---|---|
| Antifungal activity test | 100 ppm | 86 | 77 |
| | 12.5 ppm | 80 | 74 |

In a wide range of concentrations, the compound I-18 exhibited superior antifungal activity against the citrus blue mold fungi (Penicillium italicum), compared to the control compound C.

### Test Example 8: Comparison Test of Activity

A control compound D represented by General Formula D below was prepared.

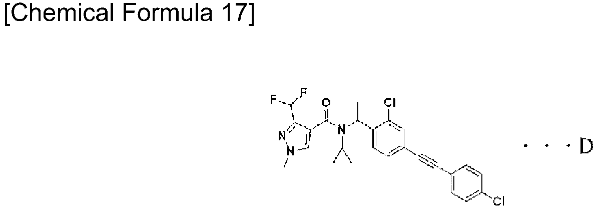

For the compound I-87 and the control compound D, the antifungal activity test against wheat fusarium head blight (Fusarium graminearum) was performed in the same manner as in Test Example 1 except for changing the concentration to a predetermined concentration. The results (mycelial growth inhibition rate (%)) are presented in Table 8.

### [Table 8]

**(Table 8)**

| Test method | Concentration | I-87 | Control compound D |
|---|---|---|---|
| Antifungal activity test | 100 ppm | 76 | 61 |
| | 12.5 ppm | 72 | 57 |

In a wide range of concentrations, the compound I-87 exhibited superior antifungal activity against the wheat fusarium head blight, compared to the control compound D.

### INDUSTRIAL APPLICABILITY

The pyrazole derivative according to an embodiment of the present invention can be suitably used as an active ingredient of an agricultural or horticultural fungicide and an industrial material protectant.

## Claims

1. A pyrazole derivative represented by General Formula (I), or an agrochemically acceptable salt thereof or an N-oxide thereof: 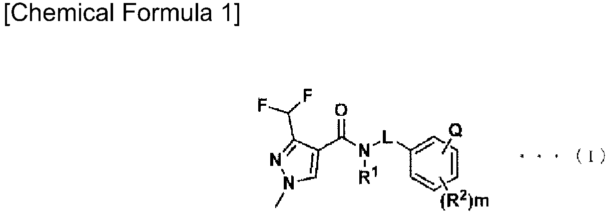 where,
Q is X-A-B;
X is a C₂-C₈ alkynylene group, a C₂-C₈ alkenylene group, or a C₁-C₈ alkylene group;
A is (i) -O-, -SO₂-, -COO-, or -NR^{Q}-, or (ii) a single bond or a linear C₁-C₄ alkylene group,
R^{Q} is hydrogen or a C₁-C₄ alkyl group,
B is a phenyl group, a benzyl group, a pyridyl group, a naphthyl group, a quinolinyl group, a pyridylmethyl group, or a tetrahydropyranyl group;
B is unsubstituted or substituted with n R^{B} substituents,
when A is (i), R^{B} is a halogen group, a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group;
when A is (ii), R^{B} is a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group;
n is 1, 2, 3, or 4, and when n is 2 or more, the plurality of R^{B} substituents are each independently selected;
R² is a halogen group, a C₁-C₄ alkyl group, a C₁-C₄ alkoxy group, a C₁-C₄ haloalkyl group, a C₁-C₄ haloalkoxy group, a C₁-C₄ alkoxy-C₁-C₄ alkyl group, a cyano group, a nitro group, an amino group, or a pentafluorosulfanyl group;
m is 0, 1, 2, 3, or 4, and when m is 2 or more, the plurality of R² substituents are each independently selected;
R¹ is hydrogen, a C₁-C₄ alkyl group, a C₃-C₆ cycloalkyl group, a C₁-C₄ alkoxy group, a C₁-C₄ haloalkyl group, or a hydroxy group; and
L is a C₁-C₆ alkylene group.

2. The pyrazole derivative, or the agrochemically acceptable salt thereof or the N-oxide thereof according to claim 1, wherein the pyrazole derivative is represented by General Formula (II):

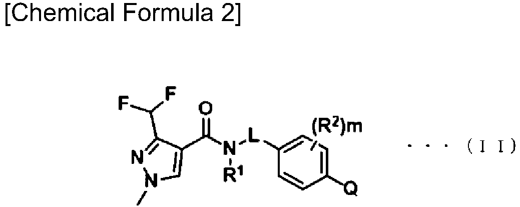

3. The pyrazole derivative, or the agrochemically acceptable salt thereof or the N-oxide thereof according to claim 1, wherein,
in General Formula (I),
X is a C₂-C₈ alkynylene group;
A is (i) -O-, -SO₂-, or -NR^{Q}-, or (ii) a single bond;
when A is (i), R^{B} is a halogen group, a C₁-C₄ alkyl group, a C₁-C₄ alkoxy group, a C₁-C₄ haloalkyl group, a C₁-C₄ haloalkoxy group, or an amino group;
when A is (ii), R^{B} is a C₁-C₄ alkyl group, a C₁-C₄ alkoxy group, a C₁-C₄ haloalkyl group, a C₁-C₄ haloalkoxy group, or an amino group; and
R² is a halogen group.

4. The pyrazole derivative, or the agrochemically acceptable salt thereof or the N-oxide thereof according to claim 3, wherein,
in General Formula (I),
A is -O-;
B is a phenyl group, a benzyl group, or a pyridyl group;
R^{B} is a halogen group or a C₁-C₄ alkoxy group; and
R¹ is a C₁-C₄ alkoxy group.

5. The pyrazole derivative, or the agrochemically acceptable salt thereof or the N-oxide thereof according to claim 1, wherein,
in General Formula (I),
when A is (i), n is 1 or 2;
when A is (ii), n is 1; and
m is 1 or 2.

6. The pyrazole derivative, or the agrochemically acceptable salt thereof or the N-oxide thereof according to claim 1, wherein,
in General Formula (I),
L is a branched C₁-C₆ alkylene group.

7. An agricultural or horticultural chemical or industrial material protectant comprising:
the pyrazole derivative, or the agrochemically acceptable salt thereof or the N-oxide thereof described in any one of claims 1 to 6 as an active ingredient.
